(19) Europäisches Patentamt
European Patent Office
Office européen des brevets

(11) EP 1 577 399 A2

(12) EUROPEAN PATENT APPLICATION

(43) Date of publication:
**21.09.2005 Bulletin 2005/38**

(51) Int Cl.⁷: **C12Q 1/70**

(21) Application number: **05003256.4**

(22) Date of filing: **16.02.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **17.02.2004 IT MI20040258**

(71) Applicant: **Universita' degli studi di Bari
70121 Bari (IT)**

(72) Inventors:
- **Barbarossa, Loredana
70121 Bari (IT)**
- **Di Serio, Francesco
70121 Bari (IT)**
- **Finnetti-Sialer, Mariella
70126 Bari (IT)**
- **Gallitelli, Donato
70121 Bari (IT)**
- **Martelli, Giovanni P.
70121 Bari (IT)**
- **Minafra, Angelantonio
70121 Bari (IT)**
- **Papanice, Mariantonietta
70121 Bari (IT)**
- **Saldarelli, Pasquale
70121 Bari (IT)**
- **Saponari, Maria
70121 Bari (IT)**
- **Savino, Vito
70121 Bari (IT)**

(74) Representative: **Gislon, Gabriele
Marietti, Gislon e Trupiano S.r.l.
Via Larga, 16
20122 Milano (IT)**

# (54) Diagnostic kit and method for detecting phytovirus infections in plant production

(57) The invention concerns a diagnostic kit composed of PCR primers and/or cloned nucleotide sequences and/or nucleic probes, for the diagnosis and identification of harmful viruses affecting the quality of nursery vegetable, flower and fruit productions among those contemplated by the Commission Directives no. 93/49/EEC of 23.06.93, no. 93/63/EEC of 5.07.93 and no. 93/78/EEC of 21.09.93; no. 93/61/CE of 2 July 1993 and no. 93/62/CE of 5 July 1993; no. 93/48/EEC of 23.06.93, no. 93/64/EEC of 5.07.93 and no. 93/79/EEC of 21.09.93 and a method for detecting plant viruses which uses said diagnostic kit.

Fig. 1

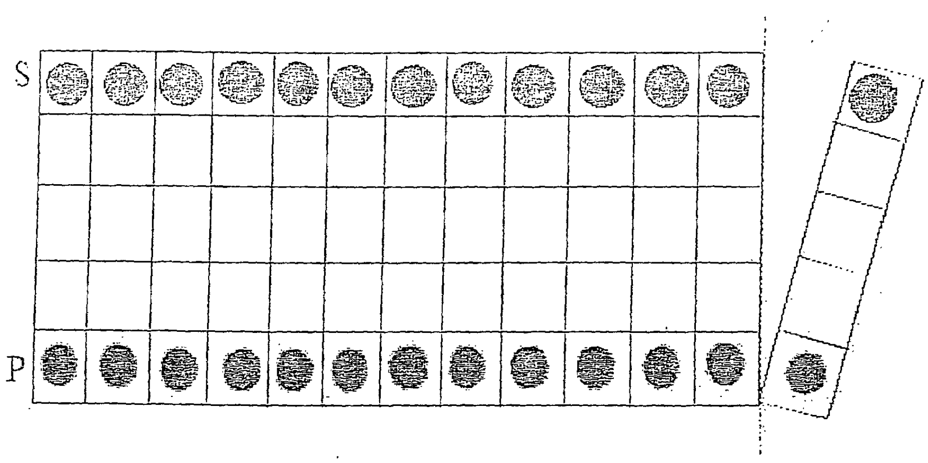


EP 1 577 399 A2

## Description

The present invention concerns a diagnostic kit composed of PCR primers and/or cloned nucleotide sequences and/or nucleic probes for the diagnosis and identification of harmful viruses affecting the quality of plant production, in particular in nursery vegetable, flower and fruit productions, and a method for detecting plant viruses (phytoviruses) which utilizes that diagnostic kit.

## PRIOR ART

Among the possible strategies for fighting plant viruses, prevention guarantees the best possibilities of success, especially if accompanied by early diagnosis on asymptomatic vegetal material, as in the typical case of nursery productions.

From the point of view of plant health, the possibility of short-term verification is today decidedly more concrete than in the past and is an element of support for nurserymen, enabling them to respect legal obligations, for example, in Italy, the restraints imposed by the coming into force of the Ministerial Decrees of 14.4.97 which set the technical standards for the marketing of nursery productions. Among other things, it is specifically required that a credited laboratory be indicated to which to entrust the performing of the tests necessary for ascertaining the plant health status of nursery productions. This process contemplates diagnostic procedures which may involve even thousand of samples and the results of which must be communicated to the client within a few days.

The diagnostic instruments available today consist essentially of: a) tests on biological indicators which contemplate long times and high costs; b) serological tests, which are very practical but not always reliable due to the poor immunogenicity of some viruses, and are not suitable for detecting viroids because they have no capsidic protein; c) molecular tests, based on the properties of the nucleic acid which guarantee very high sensitivity and versatility in use, characteristics which make them particularly suitable both for mass diagnosis and for fine diagnosis.

The latter allow the detection of specific viral sequences even in raw extracts of vegetal tissue and do not require the use of radioactive markers, which allows them to be proposed also in commercial kits that are easy to use. Further advantageous aspects that should not be underestimated in mass diagnosis are those concerning the possibility of repeating the test, even when the sample is no longer available, and to detect, in a single test, mixed infections of several plant viruses in a single sample. By applying replicas of the sample on a nylon membrane it is possible to repeat the test even a considerable time after the sample was taken, while the use of multiple molecular probes or of PCR with "degenerate" primers, that is primers that are different for one or more bases with respect to a "consent" sequence, allows the detection of infections of several similar viral species, or of species belonging to the same genus in a single reaction.

## SUMMARY OF THE INVENTION

The object of the invention is to make available a method of diagnosis and a diagnostic kit based on the genic sequences of pathogenic viral and viroidal species of vegetable, flower and fruit cultures, validated and suitable also for diagnosis for the purposes of the plant health certification required from nurserymen with relation to these pathogens, also according to the Commission Directives:

- no. 93/49/EEC of 23.06.93, no. 93/63/EEC of 5.07.93 and no. 93/78/EEC of 21.09.93 concerning the "technical standards on the marketing of ornamental plants and of the ornamental plants belonging to the genuses, species or hybrids listed in annexe I
- no. 93/61/CE of 2 July 1993 and no. 93/62/CE of 5 July 1993 concerning the "technical standards on the marketing of vegetable seedlings and material for the propagation of vegetables, with the exception of seeds"
- no. 93/48/EEC of 23.06.93, no. 93/64/EEC of 5.07.93 and no. 93/79/EEC of 21.09.93 concerning the "technical standards on the marketing of fruit plant propagating material and fruit plants intended for fruit production"

These Directives also have the objective of raising the quality standard of nursery productions, defining a new type of nurseryman, that of the "credited producer".

The method of diagnosis proposed is innovative for the specific purposes mentioned above because it aims to detect several viral species belonging to the same genus, by using degenerate primers, and is therefore usable for multiple diagnosis.

Another object of the invention is to propose the formulation of a kit for commercial use, for the diagnosis of plant viruses in nursery productions of vegetables.

## DETAILED DESCRIPTION OF THE INVENTION

These objects and their respective advantages which will be better explained below derive from the use of specific sequences on which PCR primers have been designed.

In a first instance, these primers are proposed for diagnostic purposes for detecting RNA of viruses and viroids in preparations of nucleic acid, obtained also from vegetal tissue, through RT-PCR. The amplified fragments were also cloned in suitable transcription vectors from which to generate nucleic RNA and/or DNA probes to be used for diagnosis through molecular hybridising of vegetal extracts or preparations of nucleic acid fixed on nylon membrane and chemioluminescent detection of the hybrid formed. The nucleotide sequence of the cloned fragments was also determined.

According to the present invention, the term "probes" or "nucleic probes" designates probes, riboprobes and transcripts.

According to the present invention, the term "diagnostic kit" designates a kit of a commercial or experimental type, intended for detecting plant viruses and/or viroids according to the present invention.

So according to one of its aspects, the invention concerns a diagnostic kit for detecting one or more plant viruses and/or viroids selected among the following: *Vitivirus Grapevine virus A, Grapevine virus B, Grapevine virus D, Grapevine leafroll-associated virus 4, Grapevine leafroll-associated virus 5 Grapevine fleck virus, Grapevine red globe virus, Grapevine asteroid mosaic virus, Grapevine leafroll-associated virus 7, Plum bark necrosis pitting-associated virus, Peach latent mosaic viroid, variants of Peach latent mosaic viroid responsible for calico of the peach, Plum pox virus, Citrus psorosis virus, Citrus variegation virus, Citrus tristeza virus, Olive latent virus-1, Olive latent virus-2, Olive latent ringspot virus, Olive latent yellowing associated virus, Turnip mosaic virus, Alfalfa mosaic virus, Cucumber mosaic virus, Impatiens necrotic spot virus, Tomato spotted wilt virus, Pelargonium zonate spot virus, Lettuce mosaic virus, Cucumber green mottle mosaic virus, Bean yellow mosaic virus, Celery mosaic virus, Artichoke mottled crinkle virus, Artichoke latent virus* and *Artichoke Italian latent virus* and which comprises PCR primers and cloned nucleotide sequences for the synthesis of nucleic probes.

The PCR primers, the cloned nucleotide sequences and the probes useable in the preparation of the invention kit are described in detail here below and claimed in the claims annexed to this description.

The use of the diagnostic kit of the invention for detecting infections in nursery vegetable, flower and fruit productions as required by the Commission Directives mentioned above constitutes another aspect of the present invention.

According to another of its aspects, the invention concerns a method for the detection and identification of plant viruses and/or viroids in nursery vegetable, flower and fruit productions, which contemplates the use of a particular type of diagnostic kit according to the invention.

## DESCRIPTION OF SPECIFIC EMBODIMENTS OF THE INVENTION

The following official conventions are used in the sequences of the present invention:

N=A+C+G+T; R=A+G; Y=C+T; K=T+G; S=C+G; W=A+T; H= A+T+C; B=T+C+G; D=A+T+G; V=A+G+C

### 1. Grapevine virus:

**1.1.** Degenerate PCR primers, designed on the GDD pattern of ORF1 (viral polymerase), for detecting the grapevine viruses *Grapevine virus A* (Acc.n.X75433), *Grapevine virus B* (Acc.n.X75448), *Grapevine virus D* (Acc.n. Y15892) (Martelli *et al*. 1997, Archives of Virology 142:1929-1932; Saldarelli *et al*., 1998 European. Journal of Plant Pathology, 104, 945-950). The expected amplified fragment is 363 bp.

Primer Vdpr1 : 5'-GCDAARGCDGGRCAAACCATMGCSTGY-3'

Primer Vdpr2: 5'-RAAYTCSCCSSWRAASCKCAT-3'

**1.2.** Degenerate PCR primers, designed on the gene HSP70, for detecting the viruses *Grapevine leafroll-associated virus 4* (Acc.n. AF 039553) and *Grapevine leafroll-associated virus 5* (Acc.n.AF 039552) (Routh *et al*., 1998; Phytopathology 88,1238-1243). The expected amplified fragment is 370 bp.

Primer HSP45A: 5'-GTATCTYATGTACCAACAGAT-3'

Primer HSP45B: 5'-GGTATGAACAARTTCAATGC-3'

**1.3.** Degenerate PCR primers, designed on the domain of viral polymerase (ORF1), for detecting *Grapevine fleck virus* (Acc.n. AI 3090229), *Grapevine red globe virus* (Acc.n. AF 521977) and *Grapevine asteroid mosaic virus* (Acc.n. AJ 249357) (Martelli *et al*., 2002; Archives of Virology 147 (9), 1847-1853).

The expected amplified fragment is 390 bp.

Primer RD1: 5'-CYCARCAYAARGTVAACGA-3'

Primer RDAp: 5'-CATGCANGTSAGRGGRCCRAA-3'

**1.4.** Specific PCR primers for detecting *Grapevine virus B* (Acc.n. X 75448) designed on the gene of capsidic protein (from nt 6979 to nt 7132). The expected amplified fragment is 153 bp

Primer H28: 5'-GTGCTAAGAACGTCTTCACAGC-3'

Primer BBoSe down: 5'-CGAGTAGCCCTTCGTTTAGCCGC-3'

**1.5.** Specific PCR primers for detecting *Grapevine leafroll-associated virus 7* designed on the domain of methyltransferase (ORF1). The expected amplified fragment is 186bp

Primer G23MET2U : 5'-AATGACTGTGATGTCGCTTTTAC-3'

Primer G23MET2L : 5'-TACCACTACCAGGAGGTTTATTCA-3'

**2. Virus and viroids of drupaceous plants:**

**2.1.** PCR primer for the amplification of a 290 bp fragment inside the gene of HSP70 for detecting *Plum bark necrosis stem pitting-associated virus* (PBNSPaV) (Acc.n. AJ305307). The expected amplified fragment is 290 bp

Primer ASP1: 5'-CGGTAGGGCTGTGACTACCG-3'

Primer ASP2 : 5'-GTAGTCCGCTGGTACGCTACAAG-3'

**2.1 bis** Nested PCR primer for the amplification of a 190 bp fragment inside the gene of HSP70 for detecting (PBNSPaV) (Acc.n. AJ305307). This pair of primers is used in association, in *single-tube* reaction or in double PCR reaction (*nested*), with the pair of primers described above in point 2.1.

Primer ASPn1: 5'–ACGAATCCGAGTTTCGTCGC-3'

Primer ASPn2: 5'–AGGCACTACTGACCTGTAGG-3'.

**2.2** Sequence of a 590 bp fragment of the gene for HSP70 for detecting *Plum bark necrosis stem pitting-associated virus* (PBNSPaV) (Acc.n.AJ305307) with a nucleic probe. The 590 bp fragment was cloned into a pGEM-Teasy vector (Promega) yielding the plasmid pASP3. The nucleic probe is obtained by transcription of the fragment by means of the RNA polymerase of SP6 after linearization of the plasmid with the restriction enzyme *Pst*I.

TCGAATGTTCCTCCACCGAAATCATACACAAGAGTGTAAGAAGTCGC
ACCACCAGTCTTCTCCAGTAGAGAACAGAAACCTGCAGCAGTCGGTTCAT
TGATGACTGCCCGAACACCAATGCCAAGAGCATTACAAGCAGTGTAAAC
AAAGCTGCGCTTAAATGAATTGTAGTCCGCTGGTACGCTACAAGAGGCA
CTACTGACCTGTAGGTTTGTTTGGGAAATAGTGAGTTGTACCAAACCTTT
GATATACAAGTATATCAAGTCCACAACAGGCATGACGCGAGTTAAATTTC
CGGACACAGGTCCAATCGAGACATTATATTTATCCACAATCACAGTATAT
TCTGGTTTGAGTTTGCGACGAAACTCGGATTCGTTTATGTGATTACAACC
AACCCAACGCTTAATATCTCGATAAACATCCAAACCCTCTTCGGTAGTCA
CAGCCCTACCGATGCAAAAAGTCCCATCTTTTCGCAACCCCAGAATAGTC
GGTATGAATATAGAATCCGACTCTTCAGTGCAACGTTGCTCTTATTAGCA
GGGGAGAAACATACACTGCTGAAAGTAGTTCCAAAATCAAACCC

**2.3** PCR primers for detecting *Plum pox virus* (PPV) designed on the gene codifying the helper component of PPV, strain M, (Acc.n. AJ243957). The expected amplified fragment is 893 bp.

Primer PPVHCPRO fwd 5'- GTG TGC CAA GCG ATA ATC CCT TG-3' (position 1197-1219)
Primer PPVHCPRO rev 5'- TTT GCT ATG AAC ATT GCA CCT CC-3' (position 2067-2089)

**2.4** Sequence of a 893 bp fragment for detecting PPV with a nucleic probe. The fragment corresponds to the cDNA of part of the gene for the helper component of *Plum pox virus* (PPV) strain M, obtained using the primers described above in point 2.3. The resulting cDNA was cloned into pGEM-T (Promega) yielding the plasmid pPPV-hc. The nucleic probe is obtained by transcription of the 893 bp fragment by means of the RNA polymerase of SP6 after linearization of the plasmid with *Sac*II

TTTGCTATGAACATTGCACCTCCTTTTGCTGTGGGCAAATCCACATAC
TTCGAATCACCTGAGTTACCAACAACTAAATGATTCTTTGTTGGACTTATT
ATTTCAGACTCTAGTGGTGTACCGTCTTCGTGTGTGACACAGCAACATTG
GTATACAAAATTTTCCCCTCGCTTGCTAGTGCATTCCTTTGAGACCTCAAA
ATGAATGCACTCTTCACCCAAGAGCTGTTTACGTAGTGCTGCCAGATTCG
TTGACATAACAAGGTTGCCAATGGCCAATTTGCGAATACCTCTAGGATTT
TCACGAACAATATGACGTCTGTAGCCCTCACTAACATCGATCACATCGAA
ATAATTCCTAAAGAAGCGTTTGGCATGATACTCTCTCTGTCCCCACACGA
AATTGCCATTAGTATCAAGTTGATTATCACACATAAGCTGCATATTGACG
TGTGCTTTTGATGAGATTTTGTTCCTGAAAGCCTTTACAGATCCGCTCCTA
ATGTTCTCTGTGCGATTCTTCTGATATCGCGCTAGTTCGCGCAGATGATCC
GAGGCTTCTATGTAGTCTTGTGCACTCATCATACCACCCTTAATGATTAAT
TCATTGATCTTGTTGAGATGGGAAAAAGGTGCTTCTTTGCGCTCACCTAT
CATGTGCGTGATATCCTTGAAAGCTTCATAATTCTGATTTTTCACGCGCAT
CAATTCACGATACCTCTTTAGAAAAGCGAGAACATGTGAAAATTGTGGAT
AGTTATCCATCACTGTTTTCTCATGCTGCTCAATTACTGTCGAAAATCGAT
CACGTATTTCCTGTTGTGACATGTAAGAATACTTTTGAGCACATTGCAGA
CATGTGATTTTTCCACAAGGGATTATCGCTTGGCACAC

**2.5** RT-PCR primers for detecting *Peach latent mosaic viroid* (PLMVd). The expected amplified fragment corresponds to the cDNA of the complete genome of PLMVd and it may vary between 335-338 and 349-350 bp depending on the isolates (Hernández and Flores, 1992, Proc. Natl. Acad. Sci. USA, 89: 3711-3715; Malfitano *et al*., Virology, 2003, 313: 492-501).

Primer FPLMVd-59: 5'-CTTACCTCATTGCGAGGTGCTTAGCC-3' (position 127-152 of the reference sequence published by Hernández and Flores, 1992), used in the reverse transcription reaction and, in association with the primer FPLMV-60, in the PCR reaction

Primer FPLMVd-60: 5'-GTGGGACTTTTCCTTCTGGAACCAAG-3' (position 153-178 of the reference sequence) used in association with the primer FPLMV-59 in the PCR reaction.

The primers FPLMVd-59 and FPLMVd-60 have not been described previously and they are new and original because they correspond to a relatively little variable region of PLMVd, so they are efficient on a wide spectrum of isolates.

**2.6** Sequence of a 337 bp fragment for detecting PLMVd with a nucleic probe. The fragment corresponds to the genomic cDNA of the variant PL1.1 obtained with RT-PCR from the isolate UBA.CA.P1 using the primers FPLMVd-59 and FPLMVd-60 described above in point 2.5 (the sequences of the primers FPLMVd-59 and 60 are underlined). The cDNA was cloned into pGEM-T-easy (Promega) yielding the plasmid pGPL1.1. The nucleic probe is obtained by transcription of the 337 bp fragment by means of the RNA polymerase of T7 after linearization of the plasmid with the restriction enzyme *Spe*I

CTTACCTCATTGCGAGGTGCTTAGCCTTTCTATCGGAACTGCAGTGTC
CGAATAGGGCACCCCAAGGTGGAGGGGCTGAGAGGCCACAACTCTCTCA
TAAGTCTGGGCTTAGCCCACTGATGAGCCGTTGAGATACGGCGAAACTTA
TGTGTGAAAAGAGTTTCGTCTCATTTCAGAGACTCATCAGTGTGCTAAGC
ACAGACTCTTCATCCAGAATCACTTCTGGAGGGGACCGGGTTTGAATCCC
GGGTAGACGTCGTAATCCAGTTTCTACGGCGGTACCTGGATCACACCCCC
CTCGGAACCAACCGCTTGGTTCCAGAAGGAAAAGTCCCAC

**2.7** PCR primers for identifying the variants of PLMVd, responsible for calico of the peach, containing insertions of 12-13 nt between positions 1 and 337 of the reference sequence: (Malfitano *et al*., 2003)

Primer FPLMVd-61: 5'-AAGGTGGAGGGGCTGAGAGG-3' (position 69-88 of the reference sequence), used in the reverse transcription reaction and, in association with the primer FPLMV-58, in the PCR reaction)

Primer FPLMVd-58: 5' -ATCCAGGTACCGCCGTAGAAAC- 3' (position 202-223 of the reference sequence), used in association with the primer FPLMV-61 in the PCR reaction.

The expected amplified fragment for the variants of PLMVd with insertion of 12 13 nucleotides in the loop A (between the positions 1 and 337 of the reference sequence) is about 235-237 bp, the one for the variants without insertion is about 223-224 bp. Separation on 5% non-denaturing polyacrylamide gel allows discrimination between the two types of variants.

The primers FPLMVd-61 and FPLMVd-58 are new and original because if they are used in a simple PCR reaction, they can also allow the identification of variants of atypical sequences of PLMVd cloned with standard methods, avoiding the use of sequencing.

**3. Citrus fruit viruses:**

**3.1.** PCR primers designed on the gene for capsidic protein for detecting *Citrus psorosis virus* (CPsV) (Acc. n. AF036338). The expected amplified fragment is 593 bp.

Primer cp1: 5' -GCTTCCTGGAAAAGCTGATG-3' (position 665-684)

Primer cp2: 5'-TCTGTTTTTGTCAACACACTCC-3' (position 1243-1264)

**3.2.** Sequence of a fragment of cDNA for detecting CPsV (Acc.n.AF036338) with a nucleic probe. The 600 bp fragment corresponds to the cDNA of the gene for the capsidic protein (RNA3, nt 654-1253) of an Apulian isolate of 'Navelina' VC (IAM-UBA-ps101) obtained using the primers cp1 and cp2 described above in point 3.1 The resulting cDNA was cloned into pGEM-T easy (Promega) yielding the plasmid pCPS600. The nucleic probe is obtained by transcription of the fragment by means of the RNA polymerase of T7 after linearization of the plasmid pCPS600 with *Sal*I or *Sac*I.

TCTGGTTTTGTCAACACACTCCATGTCGTAGTTGCCATTCACCATCGC
CAAAGAAGTTTGTATCATTCTGAAACCCCTGGTTCTTGTCGGTTATTATCC
TGTCGGCCATGTCAATTCTGCCATGTGGAGTGAGGCTGTAAATTATGGCA
CATGTTAACTTCAGAGTGAAGTTTTTATTGTTGGTTTTTCATCAGATAAAG
GATGAAGCCTTTTCTGATTCTTCCATTGTCCCTCCATTGTTTTCAGAAAAT
CTCTTATTGCAATTGATTTTTCAATCTTCTGATTGGTTTCTAGCAATGGCA
TCAGGGATTCAGGAGTTGCACCAACAGCAGGGGATAACTTTTGCTTGGTT
TGAAAACTGCTCGCAAACCCAGCATATCTCACAGATCGATTACCTGCAAT
ATTCAGCTTACACCTAGAGCAAACAGCACTTGGTAAGTTGTCTATATCAA
TCTTGAGAAACACCCTTGCAGGGAATTTCTTTGTTGGAGCCAATTCTTCA

CTGATTTGTGACAAACTTACATCACCATCATACAACTTCGATTTGACAAA
TTCAGGTACTTTCCTTTTGTCATCAGCTTTTCCAGGAAGC

**3.3.** PCR primers designed on the sequence of the gene for the capsidic protein for detecting *Citrus variegation virus* (CVV) (Acc.n. U17389). The expected amplified fragment is 249 bp.

Primer Sn272: 5'-TACCATTGCCTACATGACCC-3'

Primer Asn502: 5'-GCCTTCACTTCGGAAACCGTG-3'

**3.4.** Sequence of a 249 bp fragment for detecting CVV (Accession number U17389, 1592-1840) with a nucleic probe. The fragment corresponds to the cDNA of the RNA3 of an isolate obtained from Gargano Lemon (UBA-CVV -301) obtained by RT-PCR. The resulting cDNA was cloned into pGEM-T easy (Promega) yielding the plasmid pGEM CVVCP60x/3. The nucleic probe is obtained by transcription of the 249 bp fragment by means of the RNA polymerase of SP6 after linearization of the plasmid pGEMCVVCP60x/3 with *Nco*I.

GCCTTCACTTCGGAAACCGTGGTTCCGGTAGGGAAAACGTGCTGTTT
AGCACAATATTTTCCTCTCTTCACACGGATCCTATCAGGAGCGACAGGTC
CGGTGGCATTATTAACATCGAAACCATCCATAAACCCGGCATGTCCATCG
GATTTGCATACGAACCCTATCATCACCGAATACACTTTGGTGGTATCATG
GAGCGTTTTTATTTTACTTAGCTCCGAACGCAGGGTCATGTAGGCAATGG
TA

**3.5** Degenerate PCR primers, designed to obtain the amplification of the gene for capsidic protein (672 bp) of *Citrus Tristeza Virus* (CTV).

CTV-CPfor: 5'- ATG GAC GAC GA(A,G) ACA AAG-3'

CTV-CPrev: 5'- TCA ACG TGT GTT (A,G)AA TTT CC-3'

**3.6** Sequence of cDNA corresponding to the gene of the capsidic protein (Acc.n AJ518841) of a Chinese isolate of CTV, named CTV-0002 and belonging to the collection of isolates of DPPMA, to be used as a nucleic probe for detecting CTV. The cDNA, obtained using the primers described, was cloned into the "pGEM-T Easy" vector, yielding the plasmid pCTV.CP.2. The nucleic probe is obtained by transcription of the plasmid with SP6-RNA polymerase after linearization with *Sac*II.

TCAACGTGTGTTAAATTTCCCAAGCTGCCTGACATTGGTAACTACGAC

TTCATCAGCCCCTCGCTTCTTCAACAATTGTTCTTTAGCTTGTATGTACAC
AGCACACTCTAAATCAGTCAAGCCAGCTCCGGTCAAGAAATCTGCACAC
AGGTAATGATACCCGGCCGGAATCCCTGCATCTAGCGGACGTCCGCCATA
ACTCAAATTGCGATTCTGTCTACAAAAGGCTAAATAAAGGGCATCGTTAG
TCCTACCCCAGACTCGAAGGGCGTTAGCACGGTTACCGATACCCTTAGAA
TTAAACACGACGTCAGTCCAAAGTTTGTCAGACAAATCCACTTCGACGCC
CTCCCGAGTGTACGTTATACCCGTAGTGTCGTCATCACTTTGCAATGATG
AACTCTTAACCGCTAAACGATACAACATCATAGCTATGTGAAAGTCCTTA
TCTTTATCAGACAAATTAGGATACTTCCCTTTCAAAGTAAGAAACAAATC
TCTGTTCAAAGCGGCATTCTGTTGGGTACCCAACTGACGCACATCGTTCA
TCGTTATCAGAGTCGGATCGATGTGTAAGTTTACAGAACCGAAAGAAGA
CTCCGCTGCAACAACATTGTCGCCTTCTTTCGTTTCCTTGTTTTTGTTCTTC
AATTTCTTTGTTTCGTCGTCCAT

**3.7** Sequence of cDNA corresponding to the gene of the capsidic protein (accession number AJ518842) of an Apulian isolate of CTV, named CTV-0032 and belonging to the collection of isolates of DPPMA, to be used as a molecular probe for detecting CTV. The cDNA, obtained using the primers described, was cloned into the "pGEM-T Easy" vector, yielding the plasmid pCTV.CP.0032. The molecular probe was obtained by transcription of the plasmid with SP6-RNA polymerase after linearization with *Sac*II.

TCAACGTGTGTTAAATTTCCCAAGCTGCCTGACATTAGTAACTACAACCT
CATCAGCCCCTCGCTTTTTCAACAATTGTTCTTTAGCTTGTATGTACACAG
CACATTCTAAATCAGTCAAGCCAGCTCCGGTCAAGAAATCTGCACACAG
ATAATGATACCCAGCCGGAATCCCTGCATCTAGCGGACGTCCGCCATAAC
TCAAATTGCGGTTCTGTCTACAAAAGGCTAAGTAAAGAGCATCGTTAGTT
CTACCCCAGACTCGAAGGGCGTTAGTTCGGTTACCAATACCCTTAGAATT
ATACACGACGTTGGTCCAAAGTTTGTCAGACAAATCTACTTCAACACCCT
CCCGAGTGTACGTTATACCCGTGGCGTCATCATCACTTTGCAATGATGAA
CTCTTAACCGCTAAACGGTATAACATCATAGCTATGTGAAAGTCCTTATC
TTTGTCAGGCAAGTTAGGATACTTCCCTTTCAGAGTAAGAAATAAGTCTC
TGTTCAAAGCAGTATTCTGTTGAGTACTCAACTGACGCACATCGTTCATC

GTTATCAAAGTCGGATCGATGTGTAAGTTTACGGAACTGTAAGAAGACTC
AGCCGCAACAACATCGTCGCCTTCATTTGTTTCGTTGGTTTTGTTCTTCAA
TTTCTTTGTTTCGTCGTCCAT

**4. Olive virus:**

**4.1.** PCR primers designed on the gene for capsidic protein for detecting *Olive latent virus 1* (OLV-1) (Acc.n. NC001721). The expected amplified fragment is 747 bp

Primer OL1h: 5'-TTTCACCCCACCAAATGGC-3' (position 2720-2739)

Primer OL1c: 5'-CTCACCCATCGTTGTGTGG-3' (position 3467-3448)

**4.2** Sequence of a 1050 bp fragment for detecting OLV-1 with a nucleic probe. The fragment corresponds to the cDNA of part of the gene for the capsidic protein of an isolate obtained from olive. The resulting cDNA was cloned into the pSPT64 vector yielding the plasmid pSPTOLV1. The nucleic probe is obtained by transcription of the 1050 bp fragment by means of the RNA polymerase of T7 after linearization of the plasmid pSPTOLV1 with *Eco*RI

AGCCCCTCAATCTGGTGTTGGGTCCACTACCCCCCGTCCGAGACTTTC
GTCCCGTCCTCGGCGTGGATACCTGTGCAGACCCTCCCATGAGTCTTGTA
GATTGTATCAGCAAGTGATGCGACGGCCGGGGCACCACCCCCAGCCCGT
TAAACCTGTCTACCAGGATCCGATTGCTTACCGCTCTACTAAAACTTTTA
GGGATTAGCACAGTTACGTAACTCACCCATCGTTGTGTGGTTACAAATTG
ACAGTTGGGTTCACTGGTTCTATAAATTCGATTTCATACTGGCAGAACAC
CTGGCCAAAGTTGGTGGCAGCGGCTGGCCCTTGATCACTTGCAGTAAACA
GGGTAACTCCGCAGGTATCCGTTCTGGATGTTGGTGCCATGGCCAGAAAG
GTGGCCAGGGTAACATTAGGGTACCACAACTTGTCCATTCGAGCGCAGTC
CACGTCCACGACAACCATTCCAGGAATGTTGCCGAATGACGCTAAGGCC
ATAGCACCATCATACCCCGCGTAAGGCGGAAAAGTAATGGCACGGTAAC
CCTGTTGCATTTGAGTGATGCTTGTTGGCTGCGTATCGATTCTGTCGAATG
ATAGTGACATAGAAACATTGCCTTGTGTGCTGGTTGGGCAAACCGGTAGA
TAAATGAACCGGAGTTTGCGCCATCGGTACTTGCTGTACAAATCGGACAA
ACCTGCCAACCATGATAGATTGGACGGGATTACCGTGACGTAGTTGCCGA
AAATGGCACCTAGAGCGGCAGAGTTGATGGCACTTATGATTTCTGTGTTT

CTCACGACAGTCTGCTCATTAGTAGTAGTAATTCGAGGAACGTGCCCTTC
TGTAGAAATCACTCCACCAGCAACGGGTGCTATAACCGGAAGAACTTGT
AGGTTGCCCCTATTTGGGGGTGACCTTTTAGTCTTGCGAGCAGGCAGACT
TGCCATTTGGTGGGGTGAAATCCGCTTTATATAAGTGCGGACACGCTGTG
GTCCACTTTCGTAGAACCACCTCTGAATTCGACTATCCCAGTAAAAATTA
ACCA

**4.3.** PCR primers designed on RNA-3 for detecting *Olive latent virus 2* (OLV-2) (Acc.n. X76993.1). The expected amplified fragment is 690 bp.

Primer OL2h : 5'- ACGTGTTAGTCGCTGTGGTACC-3' (position 682-703)

Primer OL2c : 5'- TATGTTTGACGCACCGGAGCG-3' (position 1372-1352)

**4.4** Sequence of a 1280bp fragment for detecting OLV-2 with a nucleic probe. The fragment corresponds to the cDNA of the codifying gene for the movement protein of an isolate obtained from olive. The resulting cDNA was cloned into pSPT64 yielding the plasmid pSPTOLV2. The nucleic probe is obtained by transcription of the 1280 bp fragment by means of the RNA polymerase of T7 after linearization of the plasmid pSPTOLV2 with *Hind*III.

GACGGAGGAAAATCTCCCTCTGCACATCATGCAACAGCTTACCTTGA
GATTTATCAAATTTACTCAGATCAATTTCTGTTAAACACTTCCCCTCAAAG
CTAGGCCAAACACGGGAGAAGTCAGCCATCGGACCAGCCGGAACAACAA
CCCAATCTTGCAGACGAGAGAAAAATCTTGCCATCACCTGCAGAAACAT
GGGGGAAAAAACCTCGGTAACCCACTTCTCATGATAAGTAATGGTTTGG
GGTACCGATCGAACCGGAAAGTTCAGATCTAGAGAGAATTTCCCAGCGG
ACTTCAGCATAACTAAGTATGAGGACAAGGGGTCATCGTAACAATTCTCC
AATGAAAAGACCTTTCGACCCTTTCTATGGAAAAAATCATCCCAATAATG
ACCATCCCCCTTATCCCAGCCGCCTTCTTTCAAGAATGTCCGGAAGAACC
TATCAGCCAACCTCTTCACGTGGATCTCCTGAGGTACCTCTACCATCTGA
GGAGCTCCAACGTTTCTCTTCGAGAATGCCATTAACATTTCCTTCTTTGTA
GCAATTCGAGGCATCTCGAGACCTGTACGAATGGACGACACAGCGAAAT
CGGGTTTCCTTCTGAACCAATCTTCGTCCCGTTCGCGTAATCGCACTTGGG
TGGGGAAATCCACAGTAAAGTCATCAGTTCCACCTGCCATTGATCTGTGG
CAGGTTCACGATTAAAATGCTCGGGTGCCTTGTCTATGAAAAACTGATCA

ATTAAAGCGTCATCACCTCGCGGGAGACAGACCCTAGGGACTAAGGGTT
CCGATTGAGCTCGTGGCAACTCAACCTCTTTCAGTTTCTCACCGGAAAAA
TCCGACCGAGCGTTCAGAAGAAGGGTGGGATTAAAGTGTTCAAACAATT
CCGTACGGACTAATCTCACCATCTCTACATCCATTGGAGGCTCAGGAGCC
AAGGCTCTCTCTTGCGGATGTACGAAATGTACGTCAGAAATCAATCCTTC
TGGTTCCCAAAAATCCAGAGCAGACACGGGCTCGACCTTTCTTTCGATCT
TAATTTCAATCTCATGAGGTATTGGCACGTATTTCAAGAAACAACCAGCC
AATAATGCCAAGCCTAGGTCTTCTGTTAACCATTTCACTTTCCCGTCTTCG
AACTTAAAGGCACTAGAAATAGTGTTCGTGGCGTCAAATGTTAGACCAGT
TCCACCTTTGGCACGATAGATGAATTGCGTACCACACGAACAACTACACC
TTCCTGGGGTCACGGCATAAAAAGCTTGCATGCAG

**4.5.** PCR primers designed on the gene for capsidic protein for detecting *Olive latent ringspot virus* (OLRSV) (Acc.n. AJ277435). The expected amplified fragment is 477 bp.

Primer OLRh: 5'- TGCAAAACTAGTGCCAGAGG-3' (position 3405-3424)

Primer OLRc : 5'- TGCATAAGGCTCAGGAG-3' (position 3882-3865)

**4.7** PCR primers designed on the gene for HSP70 for detecting *Olive leaf yellowing-associated virus* (OLYaV) (Acc.n AJ440010). The expected amplified fragment is 381 bp

Primer OLYh : 5'- TTTCACCCCACCAAATGGC-3' (position 2268-2289)

Primer OLYc : 5'- CTCACCCATCGTTGTGTGG-3' (position 2648-2627)

**4.8** Sequence of a 381 bp fragment for detecting OLYaV (*Olive leaf yellowing-associated virus*) (Accession number: AJ440010) with a nucleic probe. The fragment corresponds to the cDNA of part of the gene codifying the protein HSP70 of an isolate obtained from olive. The resulting cDNA was cloned into pGEM-T easy yielding the plasmid pGEMOLYV. The nucleic probe is obtained by transcription of the 381 bp fragment by means of the RNA polymerase of SP6 after linearization of the plasmid pGEMOLYV with *Nco* I.

CGAAGAGAGCGGCTGAAGGCTCGTTCATGATATGAAAAACCGGTAA
TCCAGTACGTTCCTCCGTAGTCTTCATAAAGAATCTTTGAGTAGAAGTGT
ATTGACTTGGTACGGAAATAACTAAACCACTACAGCGAAAATTTCTTAAC

TTCTCAAAATCCCTCACAACTAAGGCAATGAAGAAAGAGACGATTTCGT
GGAGATTACGAAGTCGAGGCTGCCCAAAAGTAGGGTCAATCCTCAGTGA
AAAGTGTACCCCAGGACAAGTGACGTTGTACAGAGGTTTAAGTTTGGCA
CGTCTCTCAGAAAGCGTTTTTGAGTTGACTCCTATCCAACGTTTAAGGTCT
CTATAAACTGAGAAACCCTGTCGACCGTAACCGTCCC

**5. Virus of vegetables and of the artichoke**

**5.1** Degenerate PCR primers for detecting *Turnip mosaic virus* (TuMV) designed on the gene codifying capsidic protein of TuMV (Acc.n.AF071526). The expected amplified fragment is 473 bp.

Primer TuMV fwd 5'- AT(TC) CT(GA) TAC AC(GCT) CC(GA) GAG CA-3' (position 130-150)

Primer TuMV rev 5'- GCG CCA CGC AGT GCT G -3' (position 586-602)

**5.2** Sequence of a 473 bp fragment for detecting TuMV with a nucleic probe. The fragment corresponds to the cDNA of part of the gene for capsidic protein obtained using the primers described above in point 5.1. The resulting cDNA was cloned into pGEM-T (Promega) yielding the plasmid pTuMV.cp.5. The nucleic probe is obtained by transcription of the 473 bp fragment by means of the RNA polymerase of SP6 after linearization of the plasmid with *Apa* I

GCGCCACGCAGTGCTGCTGCTTTCATCTGGATGTGTGCTTCTCTCGCA
CGTATTGGAGTTCTAGAAGTCATTTCATAGAAATCAAACGCGTAGCGAGC
TAAGCTCATGTCGGTTAAATTGCGCTGAAGACCATATCGTGGCATGTATG
GTCGGTCTTGGTTACGCTTTACAATGTACGCTTCAGCTACGTCACTGAAA
TGGGCCATTATCTGCCTAAATGTGGGTTTGGCGTGGTCAATGAGCGGTTT
GATTGGAAATTCCACCTGATCGTCGCCGTCCATCATCACCCACATTCCGT
TTATATTTGGGGAAGTTCCATTCTCAATGCACCAGACCATTAAACCATTA
AGAATGATTTGCATTTTATCCTCCGTTAGTTCATAGTCAGCCATGACGCCC
TCAAACCATGTATTGAACTGCTTCTGTGTTGAGCGCGTGTTGGATAAGTC
TGTCTGCTCTGGAGTGTACAGAAT

**5.3** PCR primers for detecting *Alfalfa mosaic virus* (AMV) designed on the gene codifying capsidic protein of AMV, strain 425, Madison (Acc.n.Y09110). The expected amplified fragment is 751bp.

Primer CPAMV1 fwd 5'- TCC ATC ATG AGT TCT TCA C-3' (position 1187-1206)

Primer CPAMV2 rev 5'- AGG ACT TCA TAC CTT GAC C-3' (position 1919-1938)

**5.4** Sequence of a 751 bp fragment for detecting AMV with a nucleic probe. The fragment corresponds to the cDNA of the whole gene for capsidic protein obtained using the primers described above in point 5.3. The resulting cDNA was cloned into pGEM-4Z (Promega) yielding the plasmid pAMVDa. The nucleic probe is obtained by transcription of the 751 bp fragment by means of the RNA polymerase of SP6 after linearization of the plasmid with *EcoR* I

AGGACTTCATACCTTGACCTTAATCCACCCAGTGGAGGTCAGCATTA
AATGACTTTAGCATCCCAAATTAATGGGGTACGTCAATGACGATCAAGAT
CGTCAGCTTCGTCGAGCAGCCCCACAGTAATCAAACTGCGGAGGGGCCC
TGCTCGCGGGAACGCCTTTCTCTCGACCCAAACTTCGTTGAATCGGTATG
AGGGATTTTTAGATCCCTCAGGCTGCGCGTCGAAGTCCAGACAGAGGGCT
ACGGCATAGGAATGCTTGAAATTTTGATTTTGCATTCCCGCATGGGTAGG
AGCTGTGAAGACCAACTCAAACCCGAACTTCTCATTAGCGTGAAACACG
CCTTGAGGAAATTCCTGCATGGGATTACCATGCGCAACGGCCCTACCATC
CTCAGTCGTCACGTCATCAGTGAGACAAAAGGTGCCGGCATGGGACGGT
GTTATGGAAAACACCATCCTGTAAATCAGATCCTCTTCGAGGATCCGAGG
TCCCGCGAAATCCTTCAGAAAACTGTAGAGGAATCTCACGCCGAGCCCAT
TAAAAGAGCTCAGACTCAGAGGGGTCCCGAGGCTTTGCCACACGCAGCC
CGTCTGTGGCAGTATAGTATTCGTCGGTTTTACAACCGGGACTTTCAACG
CCGGAGGCTTCGGCAGTTGAGCTTTGCGCAAGGCAGCATAGTTCTGAGA
ACGTTTAGTAGGTTTACCAGCTTTCCCACCAGCTTTCTTTTGTGAAGAACT
CATGATGGA

**5.5** PCR primers for detecting *Impaliens necrotic spot virus* (INSV) designed on the RNA S of INSV, non-structural protein, (Acc.n.X66972). The expected amplified fragment is 631 bp.

Primer INSV5' fwd 5'- TGG GGA ACA ACA TCT TCG GG-3' (position 110-130)

Primer INSV3' rev 5'- CAG AAG TTG CAG ACA TTG CC-3' (position 722-741)

**5.6** Sequence of a 631 bp fragment for identifying INSV with a nucleic probe. The fragment corresponds to the cDNA of part of the RNA-S gene (non-structural protein) obtained using the primers described above in point 5.5. The resulting cDNA was cloned into pGEM-T (Promega) yielding the plasmid pINSV-NSP. The nucleic probe is obtained by transcription of the 631 bp fragment by means of the RNA polymerase of T7 after linearization of the plasmid with *Sac* I

CAGAAGTTGCAGACATTGCCAGTGATTTTACAGCCAAAGTGTTTACT
GTCCTGTTGTTGGTCTGGGATTGGTTAAAAACAGGCTTAAGTGTGTACAG
CCATTCATGAACACTTCTGGTAGGAGAAAGAACACTGACTTTGCCCATGG
ATTGGTTGTTTGAATACTTGACATCAAAAAGGACTTCTTTGACACAAGAC
AATGATCCTTTATTTGCAGCTTCAATGAAATTATTTGGAGGGAAGATGTC
AGATTTTTCAGACCGAATCTTGAAGTAATTTCAGGCACTGCTATTTGCTCT
TCAAGCATTCTGAGCAACTGAGAGGATAACACTTTGAGATGACCTTGATG
TTTAACACCGTTTGTGTTTGTTGTGTTAGATCTGACCAGAATTTCGACAAC
ATTCCCTGAAAAGGAGAAATTCAATCAATGTCATCAAAAATAGGCACAA
AACATCTCACTATAATTTCTTTTTCTTCTGTTGGGAGAAAGCCAACTTTAC
CAGTGTAACAGAAACTGGTCTTGCTCCTGGAGTCAGAATAGAGTTGAGCT
TCGACCAACTTCTTTCCGGAAGATTGATCATGAATCCAATAACTATCTAC
TACTGCTTTACCCGAAGATGTTGTTCCCCA

**5.7** Degenerate primers for detecting *Cucumber mosaic virus* (CMV) designed on the 5'-terminal end of the RNA-2 of CMV-Fny (Acc.n.D00355). The expected amplified fragment is 637 bp. The fragment allows the identification of the subgroup to which belong the isolates of CMV IA,IB and II after digestion with the enzyme *Mlu*I

Primer RNA2-5' fwd 5'- GTT TAT TTA CAA GAG CGT ACG G-3' (position 1-22)

Primer RNA2-3' rev 5'- GGT TCG AA(AG) (AG) (AT)A TAA CCG GG-3' (position 618-637)

5.8 Sequence of a 637 bp fragment for detecting CMV with a nucleic probe. The fragment corresponds to the cDNA of part of the gene for polymerase protein obtained using the primers described above in point 5.7 The resulting cDNA was cloned into pGEM-T (Promega) yielding the plasmid pGEMCMV-2a. The nucleic probe is obtained by transcription of the 637 bp fragment by means of the RNA polymerase of T7 after linearization of the plasmid with *Sac*I.

GGTTCGAAAGAATAACCGGGTACATCGCGAGACGAGATATCTATCTG
AGCGTCGTCGGCTTCACACTCTTCACTGTAGTAGAAATCAGATTTAGTGT
AAATAGCCGCGACCAGGTCTTCAAAACACTTCATGGTTCGGTGGGATGTT
CTAGGTGCCAACAACAAAGCTCTGGCCATAGCAATGCTGGAAATAAAAG
AACATTTATTAAACGCAGGGCACCATAGTTGTCCGATACGCATGGGTTTG
ACCATCGTCTCAAAAGCGACAAGGAGCTCATCATCAGATTTTTCAAGGTA
ATCTTCAGCACCATAAGTTACAAACTCATCAGACACCGCTTCAGCGGGAG
CTCCGTCAGGAGTTCGGAGGGAATGCGCGTCCTCTGTGACACTCTCGCTG
ACATCCACAGCGGGTAGGGGCCTGTAATTACGACAGGCCGCAGCAGCCT
CTTCGCGTTGCTCAGATCGCAAACGTTCCACATCCTCGGGAGTGTCGACA
CCGTAACTGCCGTTCAAAAGATTGGCTAGTGAGAATGCGGGGGCAGGGA
AAGCCATAGAAAAGATACTAGAAAGAAAAGATTTAAGTCTAGGGAGTTT
TACAGGGGAGGCAGGGGTTGAACCGTACGCTCTTGTAAATAAAC

**5.9** PCR primers for identifying *Bean Yellow Mosaic Virus* (BYMV) designed on the gene codifying the capsidic protein of BYMV (Acc.n.AB041972). The expected amplified fragment is 705 bp.

Primer BYMVCPsens fwd 5'- ACA CAA GCA CAG TTT GAA GC-3' (position 277-296)

Primer BYMVCPanti rev 5'- AGA TCA AGC TCA CAC GAG G-3' (position 963-981)

**5.10** Sequence of a 705 bp fragment for detecting BYMV with a nucleic probe. The fragment corresponds to the cDNA of part of the gene for capsidic protein obtained using the primers described above in point 5.9. The resulting cDNA was cloned into pGEM-T (Promega) yielding the plasmid pGemTBYM.4 . The nucleic probe is obtained by transcription of the 705 bp fragment by means of the RNA polymerase of SP6 after linearization of the plasmid with *Apa* I

AGATCAGGCTCACACGAGGCAACCTCACTAACTGCTTACCCTGTCAG
AGTAGAGAGAATGATACACATACTGAATTTAAAATGCTGGAGGTGAAAC
CTCACTAATACATAGTATTAAGTAATGTAACGCCAAATTATAGAGAATTT
AAAGACGGATACTCTAAATACGAACACCAAGCATGGTGTGCATATCACG
ATTGACTCTCCTGCTGTGTGTCTCTCTGTGTTCTCCTCGTCTGTTCCAACAT
TGCCATAAGTCCAAATAATCTAGTTGACTTGCCCCTAACTGCTGCCGCCT
TCATTTGCATGTGTGCTTCTCTAGCACGCACAGGAGTTCTTGAAGTCAGTT
TGTAGAAGTCAAAAGCATACTAGCCAAACCATAATCGGTCAAGTTCCTTT
GAAGTCCATACCGTGGCATGTACCTTCTGTTGCATTCCTCTTTTCAATGTA
GGCTTCTGCAACTTCTGAGAAATGCGACATTATTTGGCGGAATGTTGGCT
TTGCATTGTCCAAGATGGGTTTTAGAGGGTATGTCACCTGTTCCTCTCCAT
CCATCATTGTCCATTCACCTTGTAAATCTCCTGATGTGCATTTTCTATGCA
CCACACCATAAGGCCATTCAAAATAATTCCCATTTGTGAATCCTCAACCT
CATATGCTTGCTTGACACCATTGTACCACGCTTCAAACTGTGCTTGTGT

**5.11** PCR primers for identifying *Tomato spotted wilt virus* (TSWV) designed on the non structural gene of RNA-M of TSWV (Acc.n.AB010996). The expected amplified fragment is 679 bp.

Primer TSWVNSM fwd 5'-GA(AG) GAA ACA TCT TCC TTT GG-3' (position 284-303)

Primer TSWVNSM rev 5'- CCT CTT CTT CTT CAA CTG ATC-3' (position 942-962)

**5.12** Sequence of a 679 bp fragment for detecting TSWV with a nucleic probe. The fragment corresponds to the cDNA of part of the gene for non structural protein (NSM) obtained using the primers described above in point 5.11. The resulting cDNA was cloned into pGEM-T (Promega) yielding the plasmid pGEMT-Dat2. The nucleic probe is obtained by transcription of the 679 bp fragment by means of the RNA polymerase of SP6 after linearization of the plasmid with *Sac* II

CCTCTTCTTCTTCAACTGATCTCTCAAGATTTGAGCTCAATTCTTTAAG
CTGTTTTTTAATCTGCTTCTCACTGTTTCCTTTAGGAATTATCAGCTTGCA
AGCCTCAATGAATGCTTGAGATCTAGCTCTAATGGCTCTGTTTAGAGGTA
TGACCATGCAACTTTTATCTTTATCAGCTCTGGGTGAATCACAAAACTCTT

TTGTCCAAGAATACATGACACTACCAAAAGAAACCCCCTTCTTGTATTCT
TGGCTGCACATCAAATGCAGCTGACAGCAGTTTTCTGGAGTGTTATTCAT
TTTCGGAATAGACCAGTTCAGATAAAAGACAAAACATATAGGATCAGTT
ATAGTCCCCTGACCCTTCAGAATGACTTGCTTTTCAGATGGCATGTTGGG
ATCGACCAGAGCAACCACAAGTTTTCCTGTAGGGTTTGGTATAGTGGGGC
AGACCCATATCACAATCCTGGAAATCATCATGTATTGTTTTCTGCTGTCCC
AAGTTGGACAGATCTTGATGACCTTATTTGCGTTTTGCTTCCCGTTGCCAA
CAAAGAGATCATTTTTCCAGTTTGAGATATGATGGTTTGTATCTACTATCA
TTCTTGCAGAAAGATCATAACCCTCTGATTCTGTGATAGAATCAGACTCA
TAGGTTCCAAAGGAAGATGTTCCTTC

**5.13** PCR primers for detecting *Pelargonium zonate spot virus* (PZSV) designed on the gene codifying capsidic protein (Acc.n.AJ272329). The expected amplified fragment is 626 bp.

Primer PZSVcp fwd 5'- ATG CCC CCT AAG AGA CAG-3' (position 1619-1636)

Primer PZSVcp rev 5'- AC AGA GGT ATA TAC TCT GC -3' (position 2225-2244)

**5.14** Sequence of a 626 bp fragment for detecting PZSV with a nucleic probe. The fragment corresponds to the cDNA of the gene for capsidic protein obtained using the primers described above in point 5.13. The resulting cDNA was cloned into pGEM-T (Promega) yielding the plasmid pPZScp. The nucleic probe is obtained by transcription of the 626 bp fragment by means of the RNA polymerase of T7 after linearization of the plasmid with *Sac* I

ACAGAGGTATATACTCTGCTTGGCCAATCTTTTCGTGCTCTACGAAAA
TCTTGACAGTAGCGACCGGAGAGTTGTTGCCCACTATAGCATAGTCCGTA
GCAAACAGATACACCCTCAATTTAGCAAGGTGTTCAACTGTCTTCCCCGG
ATAGTGGTAAATAACATGCCCTTTACCTTCCCTCCCAGTAATTCTGACTGC
ATTACTAGGAAAAGACCCCACAGAGGGGGCGACCCATCTATCACCCAAA
GCACACCAAAACTTGTGCGCCTCAGGGACAGTAGGATCAAGGAGCACAA
CAACAGCAACTCTATTTAATCTCAAGTTAGGCGACTCGAGAAGAGCATCC
GGAACCGTAAGACTCCGCTCTGCCGTAGCGAACCCAGTATGAGTCCCAA

ACGTTAGAGACGGATCAAACGTCACCTCCGTGGCGGTAAAGCCAGGGAT
GAGCTTGAGCCTACTTTCAGATTCCGTGATATCAGCCCAGCTGGTACTGG
GAGACCTTTCAACAGACATAGAGAGCCCGGAAAATTCACGGGCGAGCTT
AGCGAGTGCCTGCTGGCGACTGCGCCTCGCGCGGTTTTGTCTAGCTTTGC
GCGTCTCAGTGTTCTGTCTCTTAGGGGGCAT

**5.15** Primers for detecting *Lettuce mosaic virus* (LeMV) designed on the gene codifying capsidic protein (Acc. n.AJ306288). The expected amplified fragment is 837 bp.

Primer LeMV fwd 5'- GTA GAC GCA AAG CTT GAT GC -3' (position 9035-9054)

Primer LeMV rev 5'- TTA GTG CAA CCC TCT CAC GCC -3' (position 9851-9871)

**5.16** Sequence of a 837 bp fragment for detecting LeMV with a nucleic probe. The fragment corresponds to the cDNA of the gene for capsidic protein obtained using the primers described above in point 5.15. The resulting cDNA was cloned into pGEM-4Z (Promega) yielding the plasmid pLeMV. The nucleic probe is obtained by transcription of the 837 bp fragment by means of the RNA polymerase of T7 after linearization of the plasmid with *Eco*RI

TTAGTGCAACCCTCTCACGCCTAAGAGAGTATGCATATTCTGATTTAC
ATCTGCGGCTGTGTGCCTCTCCGTGTTCTCTTCCTGGGTTGAACCGCCTCC
ATCCATTCCAAACAGTCTGTTCTGTGTTCCCACTAGAGCAGCTGCCTTCAT
TTGATTGTGCGCCTCTCTTGCCCGATTTGGGGTCGCTGATGTTGTTTCGTA
GAAGTCGAAAGCATAGCGAGCTAGCCCCATATCGTTCAAGCCTCGTAGC
CGTCCGTATCGTGGCATATACGGTTTCTTCTTGTTTCTCATCTCAATGTAC
GCCTCGGCTGCGTCACTGAAGTGGGCCATTATCTGGCGAAACGTGGGTTT
CGCGTGTTCGATGATGGGTTTTAGAGCATATTCTACTTGTTCTTCACCATC
CATCATCACCCATGTTCCATTTATTTCGGGGATGTCCCGTTTTCTATACAC
CAAACCATCAATCCGTTCAGAATTAATTGCATGCCACTATCATCCACATC
GTAGTCATTCTTAACCCCGTCGTACCATGACTCGTACTGTTTCTGGGTTGC
ACGTGTGTTCGATATGTCTCTCTGGTTTGGTTCGTACTCCAGCAAATGATC
AAGGTTTAAAGCCACTTTACCTCGGATCATTGGTAACTTCATTTTCTGTGT

GATTGCCTTTGTACGAGGTATGGTGTGTTTACCATGTAGCCCTGCGTTGAT
GTCGTCATCCTTCTTCATCTGCCCAGAACCACTTCCTTTCTCTGTGATGAT
ATTATCCTTTGGATCCGCTGAGTTCTTTTGCTTATCATCAGTTTTACTGCCT
TGGCCTGCATCAAGCTTTGCGTCTAC

**5.17** PCR primers for detecting *Cucumber green mottle mosaic virus* (CGMMV) designed on the gene codifying the capsidic protein of CGMMV-NS strain (Acc.n.AJ243831). The expected amplified fragment is 476 bp.

Primer CGMMV fwd 5'- CTT ACA ATC CGA TCA CAC -3' (position 33-50)

Primer CGMMV rev 5'- GCT TTC GAG GTG GTA GCC -3' (position 493-510).

**5.18** Sequence of a 478 bp fragment for detecting CGMMV with a nucleic probe. The fragment corresponds to the cDNA of part of the gene for capsidic protein obtained using the primers described above in point 5.16. The resulting cDNA was cloned into pGEM-T (Promega) yielding the plasmid pCGMMVcp. The nucleic probe is obtained by transcription of the 478 bp fragment by means of the RNA polymerase of SP6 after linearization of the plasmid with *Sac*II.

GCTTTCGAGGTGGTAGCCTCTGACCAGACTACCGAAAACGCGGCTTC
AAATGAAGCCCTATCGTAAACATCAAAACCCTTAGAAATAGACTCTATTA
AATTATCTATCTCAGCCCTAGCGGCCGTAGACGCGTCATCAGTACGCTTT
ACAGCGTTAAGCGACTCAGCAGTCGTAGGATTGCTAGGATCTACAACCTC
AATGACCCTATTACGCGTATCCGTGGAGCTGAGAAGCGAAACGAAGATA
GGCCTCAACACAGGACCGTTGAGGAAAGCGTAAAAACCCGCATCTGGGA
ATCTAGAATTAATATCTACGACAGACGAGGGTAACGCAGACAGGGACTC
GCGGAAAGAATCTCTTCCCGCTTGAGTCTGGAAAGCGGTACCTTGTGAAG
CAACTAGAAAATTAAGTAAAGTCCTGACGGGAACATAAGAAGCACTAAA
CGCAATAAGTTTGCTAGGTGTGATCGGATTGTAAG

**5.19** PCR primers for detecting *Artichoke mottled crinkle virus* (AMCV) designed on the gene codifying capsidic protein (Acc.n.X51456). The expected amplified fragment is 1349 bp

Primer AMCV fwd 5'-ATG GCA ATG GTA AAG AGA AA -3' (position 79-98...)

Primer AMCV rev 5'- CTC GGA CTT TCG TCA GGA AGT TTG AA-3' (position 1402-1427...)

**5.20** Sequence of a 900 bp fragment for detecting AMCV with a nucleic probe. The fragment corresponds to the cDNA of part of the gene for capsidic protein obtained using the primers described above in point 5.19. The resulting cDNA was cloned into pGEM-4Z (Promega) yielding the plasmid pAM1. L The nucleic probe is obtained by transcription of the 900 bp fragment by means of the RNA polymerase of SP6 after linearization of the plasmid with *Hind*III.

CTCGGACTTTCGTCAGGAAGTTTGAACCCAGAAGTGGTACCTCCTGA
TCCTCCATCCCAACGTTCACCATTAGCTTGTTCCCTAGCGTCGTTTCCTTG
TATAACTCGCTCCATGGTTTATTGACTTGTTCGTATTCAGTATCCATGAGC
TGGTCTAGTTCAAGAGGCCCCTCACAGGACTCACTAAATTAAAGAGAGA
TCGTTCTGTCGCGTAGCACGATTGCACTGTATCGTGGCAGATGTTATACC
AGTGGTAGTAAACGTGATAACGGACGGTAGGTTTGAGACAGTGCAATTT
ATGAAAAATGAACTGCTCGTTCCAACATTGTCCACAACTGTAATGCTGTT
CACGTTGACCCCACCTGTCAGGCCGAGCACTGTTGAAGTTAGACAACGAT
ATGCTCCAGAGATGACAAACGTGCCAGTGGCACAGATAGTCATTGTAAG
TACAGTGGGCGTCCTGGTTAACAGGATGTACCCGGGTCCGGATGCTGTGA
CCGGTGTGCCAGCAATGTCGAGCCTTCTAGTGCTAATTAGAGTATTCGTA
GGTTGAGGGAAATATAACGTAACACTGTAGGAGATGAAGATATCCCCAA
CAGCATTAGTTCCAGCGCCACCGTATGTAGCCACGCCCAACTGTCCTAGA
TCGATAAGTTTGTGATCTAATGTGGAACTGTCATCACAAAATCTCTTAAT
CCTATCGGTAGGAACCCAGAGCGCACGTTCAGCCCAGGGGGCTGTCTCTG
CTAGTACGCTGTAGTTTGCCAACTCAACCCTATCAGCAGGTTCAGGATCT
TCCGAATCTTTATCAAAGTACATAGCCACTCGCCCCACCTCAGTGGTTGC
GCACAGAGGGACATAATGTAGCAAAACGCTGTTGAACGAGTACTGATCA
AAGTTGGAGCGTATCGCGGGCAACCATGAGAACAAAGTACCATTCAACG
GGTTGAGCTGTAACAAATTGCCAACAATTCCCCCGTTTACCTGGAAACCT
GTGGACATATTCACTTGTGATAAGTATTCACGGTGCGTAACCGTCACAGA
CCCAGACGTCTTACCGGTAAACTTAGGCTTACTCCCGGTAAGTTGTCTCG
TAACTGCAACCGGGGCCATAATGGCCCCACCTGTACCACCTACATGCTTA

ATCTGCTGCTGTTTCTTACCTCCTCGCTTCTTCACTGCTTTGTACACCTTCT
GTCCAACATCGACGACCTTTCCGACAATAGCCGCCCCGTTGTTCCTTACA
AATCCCTGCAGGGCTGAAGCCCCTGCGGCCGCTCCTAATGCCATTAACTG
CTTCATGCTAACTGGGATAAGCCCAGTGTTATTATTTCTCTTTACCATTGC
CAT

**5.21** PCR primers for detecting *Artichoke Italian latent virus* (AILV) designed on the gene for capsidic protein (Acc.n.X87254). The expected amplified fragment is 760 bp.

Primer AILV fwd 5'- ATT CAC TAG TCC CTA TTT AG -3' (position 175-194)

Primer AILV rev 5'- GGT CTG GGG TGC CCG TGG CG-3' (position 924-943)

**5.22** Sequence of a 760 bp fragment for detecting AILV with a nucleic probe. The fragment corresponds to the cDNA of part of the gene for capsidic protein obtained using the primers described above in point 5.21. The resulting cDNA was cloned into pGEM-T (Promega) yielding the plasmid pGTAILV.cp.6 . The nucleic probe is obtained by transcription of the 760 bp fragment by means of the RNA polymerase of SP6 after linearization of the plasmid with *Apa*I.

GGTCTGGGGTGCCCGTGGCGCACTCAATGGCATAATCCAAAAGCGCG
CACCACTATCACCAAAATTGGCTGTGGCACAACAGGCAGCTTGGATAAC
AGCTCTCCAGTCCAGCCCCATCAATGTCAATGTGTGGAATTTGGGCCGTC
TGCACTGCGGTTGGCTGTGTTCCCAAAGAAAAACACGAAGCTTACTTGTT
AACAAACAACTGGAAGTCGAGTGTACACGAAACCTCAGCTTTCCCCCAA
TTCCCAAGAAGTGGGACAAGAGTCCTGCAGAGTAGGAATAGGTTTCCCT
CTTTGTTGAAGTAATAGACTTTTCAGCAAAGGAAAGAGGGAAATTGGTG
GTAGCAGTTGTACCAATAGCAAGTTGTTGGGGTGCCAACAGTAGATCAA
GATCCGCCAAGTGCGTGGACTCGACGGATAGGGTTAAAATCTTTCTCCGT
AGCAAGTCCTCGGACGAAACTTTCTCCTGTGGATATCCTTCAACAACATA
TTGAACATCCGCGGAACACGGCATTTGGGCCGAGGAGGCAGCAATGATC
CAAACCTTGGGCTTCATAAACCCTGGTCCGGCAAAGTTCAAACTCTGGCC
CCCCTCTCTGTGGAAATTGAGGATCCACGTGCTGGTAGTATTGTCTTTCA
AAATGTGCACGTGTGGACTCAAGTGACGGATGCTTTCCAATTCCACAGTT
GTGGTAACGTCCGTGGGAATATGGCCATAAGCATCAAAAACATACCAGA

TGGCCATTCCTAAATAGGGACTAGTGAAT

**5.23** PCR primers for detecting *Artichoke latent virus* (ArLV) (Acc.n.X87255). The expected amplified fragment is 580 bp

Primer ArLV fwd 5'- TTG TTC ATA AGG GAG CGC GT -3' (position 10-29...)

Primer ArLV rev 5'- CTCAAG CTC TCG AAC TAA CT -3' (position 489-508.)

**5.24** Sequence of a 580 bp fragment for detecting ArLV with a nucleic probe. The fragment corresponds to the cDNA of part of the gene for capsidic protein obtained using the primers described above in point 5.23. The resulting cDNA was cloned into pGEM-4Z (Promega) yielding the plasmid pAL37-2. The nucleic probe is obtained by transcription of the 580 bp fragment by means of the RNA polymerase of T7 after linearization of the plasmid with *Eco*RI.

CTCAAGCTCTCGAACTAACTGAACTACAGATGACTTTAGGGCAACGT
CATCCACTTTGCAAAGGTGTTGGGGTCTGTTGTACTTGAGTAAATCCTTCC
AATACGCTTCACGGTTCAGTGCGCTTGGCATGTAGTTGTCCTCATACTTCT
CCACAACAGGATCAACCCATTCGTTGCACAACTTAAATTGCTTCCAATAG
GGATTCTCTCCTTCAAAGATATGCCTCGTGTTCAACAGCCCATTGGAGTA
GGCTAAGGTTGTGCATGTGTATCGCTAGATCAGTTGGTGGCAAGTTGCGT
TTTTCCACCACCTCGCGTGGCTTAAACAAGCCACCCCGTGCCGCACTTCT
CATTAAGTTCTCAACTGAGTAGACACTTGTGTCACGTGTAAACTGTCTGA
ACTCGATTGTGTCAATCTGCAAGTTCTTAATGCGTGCAGAGTTGTGACCA
TGCATCTCGGGTACAAATTCCCAGGCCTTAACGCGCTCCCTTATGAACAA

**5.25** Probe obtained on the 3' terminal region of the viral genome *Celery mosaic virus* (CeMV) retro-transcribed with a primer of Oligo d(T), on which the second helix has been synthesised using random oligonucleotides.
The fragment corresponds to the cDNA of part of the gene for the polyprotein of the genomic RNA. The cDNA was cloned into pGEM-4Z (Promega) yielding the plasmid pCeMV.8 The nucleic probe is obtained by transcription of the 650 bp fragment by means of the RNA polymerase of T7 after linearization of the plasmid with *Eco*RI.

ATCTCTTGAGTTAATTGTTGATAACCCCAAGATTCCACCATCGCTGCA

CAAATATCCTCCAATCTATGAGCTGGTTCACTTGATCGATCCCATTCTAGT
ATTGAGACAATTCTCTCTTCCTCAAGTTTTGGTATATATATTTCTCCCACA
CGAACTCCCTTATGTGACATGAACCATAACTCCTCCTTGTTCTCAGTTCTG
GTGGTGAATGTGTATTTGAGTCCCATCTCTCTGAAAATGTCTTCTAGCTTG
CTTGCGATGTGTTCATGATCTGGATGAAATGCTACAAGTAAGTCGTCCCC
ATTTATTAGGAATCTGCACACGTCGTCGTGTACATTTTCCTCATAGCCAA
GGAGCGTTAACGAGTACGTCATTGCCAAAACCACCATCAAAGTATTGT

**OPERATIVE METHODS**

The RNA preparations that were used as a "mould" for the synthesis of the cDNA fragments to be cloned were prepared according to standardised methods currently used in plant virology. The cDNA was synthesised by means of RT-PCR essentially as described by Minafra and Gallitelli (1995) (Methods in Molecular Biology , vol. 50: Species Diagnostics protocols: PCR and Other Nucleic Acid Methods, edited by J.P. Clapp, Humana Press Inc. Totowa, NJ) and then cloned into suitable transcription vectors according to standardised methods described by Sambrook *et al.*,*(*1989) (Molecular Cloning: A Laboratory Manual. New York: Cold Spring Harbor Laboratory).
The same RNA preparations were used to assess the diagnostic procedures with RT-PCR (Minafra and Gallitelli, 1995) and molecular hybridising (Gallitelli and Saldarelli, 1995 Methods in Molecular Biology, vol. 50: Species Diagnostics protocols: PCR and Other Nucleic Acid Methods, edited by J.P. Clapp, Humana Press Inc. Totowa, NJ9).
According to another of its aspects the invention concerns a diagnostic kit for use according to the invention which comprises one or more specific probes for the viruses described above.
These probes, of the riboprobe type, were obtained by transcription in vitro of fragments of cDNA of the viral genome cloned into suitable transcription vectors. Transcription was carried out with the DIG RNA Labelling kit (Roche) using DIG-UTP as a marker.
For use in the invention kit, each riboprobe may be used, alone or in combination, at various concentrations, generally at the concentration of 100 ng/ml. The hybridising signals may be revealed with suitable detection kits, for example with the DIG luminescent detection kit (Roche, Germany), following the manufacturer's instructions. In this case, after treatment, the membranes are exposed against radiography film the first time for a minimum of one hour and then for further 16 hours.

Examples of diagnosis using the invention kit are supplied below in the experimental part.

EXPERIMENTAL PART

EXAMPLE 1

**Preparation of a commercial kit for the diagnosis of plant viruses in vegetable plants.**

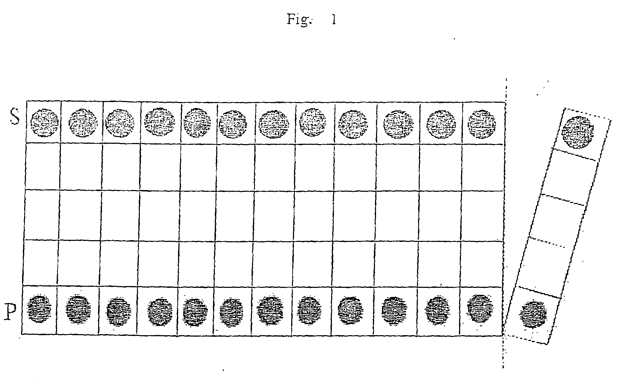

After synthesis, the riboprobes described above in points 5.2, 5.4, 5.6, 5.8, 5.10, 5.12, 5.14, 5.16, 5.18, 5.20, 5.22, 5.24, 5.25, are dried in a vacuum and kept at +4°C, in the form of a dried precipitate and included in the kit in aliquots in sufficient quantity for a multiple hybridising reaction and for as many separate hybridising reactions as are the riboprobes supplied in the kit for the hybridising of a "standard" membrane of 10 by 20 cm, useful for the analysis of about 200 samples of plant extract of 5 microlitres each.
Preferably the kit also includes a set of membranes pre-soaked with a solution of 50 mM NaOH- 2.5 mM EDTA, in sufficient number for a multiple hybridising reaction and for as many separate hybridising reactions as are the riboprobes supplied in the kit. These membranes contain in the first row juice extracts from a healthy plant and, in the last row, preparations of the same plasmidic DNA (or DNA amplified from plasmid by PCR) used for the synthesis of the riboprobes, to be used as a positive control. This arrangement, represented in Figure 1, in fact allows the use of even single portions of the membrane, depending on the test requirements.
In particular Figure 1 represents a diagram of a nylon membrane to be inserted in the commercial kit of the invention for the diagnosis of plant viruses of vegetable plants by means of molecular hybridising. The membrane (Amersham Hybond $N^+$ or equivalent), which has a height of 10 cm and a width of 20 cm, is divided into I cm squares and pre-soaked with a solution of NaOH 50 mM-EDTA 2.5 mM corresponding to the rows marked S and P are applied, respectively, negative and positive controls. The negative control is composed of 5 microlitres of juice extracted from a healthy plant in NaOH 50 mM-EDTA 2.5 mM; the positive control is composed of 50 nanograms of the same plasmidic DNA used for the transcription of the riboprobe that is to be used for the diagnosis. Membranes may be prepared containing, in P, the same plasmidic DNA (or DNA amplified from plasmid by means of PCR) or a different plasmidic DNA (or DNA amplified from plasmid by means of PCR) in each position of row P for multiple diagnosis on the same samples. The arrangement of the controls on the membrane allows their even partial use such as for the portion which appears detached on the right of the drawing. This membrane diagram may be adopted also for diagnosis of any type of pathogens.

EXAMPLE 2

**Diagnosis of viruses of citrus fruit, grapevine and olive by means of PCR**

For this test, the total RNA was purified from green leaves or from phloem of dormant cuttings kept at 4°C in plastic bags, with chromatography on silica (Foissac et al. 2000; 18th International symposium Virus and Virus-like diseases of fruit trees, July 9-15 2000, Canterbury, p. 48). Aliquots of 100-500 (0.25-0.5 in the case of CPsV) nanograms of total RNA are denatured for 3' at 100°C in the presence of random hexamers and retro-transcribed with MMLV (Invitrogen) for 1h at 42°C. One tenth of the volume of cDNA was amplified with Taq DNA polymerase (1U, Promega) and 200nM of each primer at a matching temperature of 52°C (60°C in the case of CPsV). The DNA products are highlighted on coloured polyacrylamide with silver nitrate (Minafra and Gallitelli, 1995).

EXAMPLE 3

**Diagnosis of viruses of vegetables for certification purposes**

For this test, the samples were taken from plants raised in the nursery from one to two weeks before marketing. The nursery structures should conform with those required by the Plant Health Service of the Region of Apulia for nurseries approved according to the Ministerial Decree of 14.4.97, or at any rate they should conform with those required by the Plant Health Service of the Region in which they intend to operate. For the number of samples to be taken, two levels of plant health control are considered: certification and the minimum requirements applying the M.D. of 14.4.97. For certification, each work sample is composed of 24 sub-samples of leaf tissue collected with a randomised systematic method (Barnett, 1986; Surveying for plant viruses: design and consideration. Pages 147-166 in: Introduction to plant disease epidemiology. Eds. Campbell CL & Madden LV, Wiley Interscience, NY), that is at fixed points, located at a constant distance from each other along a trace in the shape of a W. The trace may be composed of elastic thread suited to cover about 2 $m^2$ of seedlings corresponding to about 2400 plants. For the controls in conformity with the M.D. of 14.4.97, the same sampling system is followed but the 24 sub-samples are taken from batches of about 100,000 plants. In any case it is necessary to take at least one sample for each batch of plants, even less than 100,000 in size, where by batch is meant a group of plants of the same age, derived from the same batch of seed and grown in the same greenhouse. Concerning only the certification procedures, the assessment error probability (PEA) was estimated with the formula of Clayton and Slack (1988;. American Potato Journal 65: 711-723), hypothesising a uniform distribution of the infection. The optimum ratio between plant tissue and volume of the extraction solution was determined on the basis of the mean weight of 100 work samples, taken in the nursery, which was estimated at about 1.2 g. This value was considered for the preparation of an experimental work sample, on which to determine the limit of sensitivity of the method. For this purpose the most unfavourable condition was reproduced, in which only one of the 24 sub-samples was infected, mixing 50 mg of plant tissue, taken from a plant infected with one of the viruses being studied, with 1.15 g of plant tissue taken from healthy plants. For the multiple diagnosis, aliquots of 50 mg of infected tissue are mixed, taken for each of the viruses being studied, and the sample is made up to 1.2 g with tissue taken from healthy plants. The work sample thus composed was ground with an ordinary roller press in the presence of 6 volumes of a solution of 50 mM NaOH, containing 2.5 mM EDTA, and the extract applied in aliquots of 5 microlitres on nylon membranes (Hybond N+, Amersham Pharmacia) pre-soaked with the same NaOH-EDTA solution. For routine tests, about 200 samples were applied on a standard membrane 20 x 10 cm and at least 8 replicas were prepared for each sample. The positive and negative controls were represented, respectively, by preparations of plasmidic DNA containing the fragment used for the transcription of the probe and raw extracts obtained from healthy tomato plants. After the application of the samples, the membranes were exposed to UV for 5 minutes and kept at environment temperature wrapped in Whatman 3MM paper and aluminium foil; until they were used.

**RESULTS OF THE TEST CARRIED OUT**

The probes of the citrus fruit viruses used in molecular hybridizing proved to be a much more sensitive and reliable diagnosis tool than serological tests, especially for CTV, with an increase of the positive samples found (up to 70%), and for CCV, for which the conventional serological test is unreliable on account of the poor immunogenic power of the virus.

The diagnosis through molecular hybridising of the examined viral pathogens of olive makes it possible to detect 5-100 picograms of RNA per microgram of total extracted RNA. The RNA probes were validated on field material taken in different growth periods. It is possible, without reducing the sensitivity of the test, to mix two or more probes in the same hybridising mixture to highlight mixed infections.

For the grapevine and fruit trees (PBNSPaV) it was not possible to obtain positive results with hybridising because of the limitation of the viruses concerned to phloem tissues and therefore the poor overall concentration, while the application was validated for PLMVd on leaves in spring and summer.

In so far as concerns RT-PCR, on all woody hosts and for all the pathogens examined, sensitivities of fractions of a pictogram of viral RNA per microgram of total extracted RNA were obtained. For the vegetative propagation of woody species, the possibility of performing amplification tests on nucleic extracts from dormant material is fundamental, taking samples from different parts of the plant, on account of the erratic distribution of the viruses in the infected plants. On this matter it must be pointed out that it is necessary to make use of nested-PCR for detecting PBNSPaV on dormant material, whereas standard PCR does not detect any positive sample on this type of material. The specific nature of the hybridising signal or of RT-PCR is assessed by using positive and negative controls or by Southern blotting of the amplified bands.

With relation to vegetable plants, the sampling pattern used produced PEA values between 35 and 0.03% depending on whether the frequency of infection was 0.1 or 1% respectively. After collection, the samples were kept at +4°C for a variable period of one to three weeks. Even if the plant tissues did not show evident signs of deterioration, TSWV diagnosis with this method was reliable only within the first week of conservation. After that period the detection of the virus was uncertain even using plant samples without evident signs of deterioration.

The optimum ratio between plant tissue and volume of the extraction solution was 1 to 6. With a ratio of 1 to 3, the excessive presence of plant residues on the membrane often generated non-specific signals, while a ratio of I to 9 or 1 to 12 reduced the signal intensity of some viruses. In the case of work samples of 1.2 g soaked in 15 ml of extraction mixture, it was estimated that a weight of about 400 milligrams of plant tissue corresponded to 5 microlitres of extract (stain) applied on the membrane. Using the riboprobes in separate hybridising reactions and considering the most unfavourable case in which only one of the 24 plants that made up the sub-sample was infected, the sensitivity limit was about 17 micrograms of infected tissue per stain. Using the probes in a mixture the sensitivity limit was between 17 and 21 micrograms per stain but each riboprobe maintained its own specificity. Lastly the use of double exposure against a radiography plate allowed the reduction of possible false negatives even though in 90% of the cases the signal was already clearly visible after one hour of exposure. No reductions in signal intensity were observed in membranes kept at room temperature and subjected to hybridising at a distance of more than one year from preparation. The method was also applied to the checking of the state of health of nursery production of seedlings of lettuce, prickly lettuce and endive, allowing the detection of TSWV and of the lettuce mosaic virus (LeMV). Consistent infections (up to 70%) of LeMV were found in lettuce seeds, carrying out molecular hybridising on seedlings in the cotyledon stage, obtained from seeds placed to germinate on sterile substratum.

## Claims

1. Diagnostic kit for detecting one or more plant viruses and/or viroids and their variants from among *Vitivirus Grapevine virus A, Grapevine virus B, Grapevine virus D, Grapevine leafroll-associated virus 4, Grapevine leafroll-associated virus 5, Grapevine fleck virus, Grapevine red globe virus, Grapevine asteroid mosaic virus, Grapevine leafroll-associated virus 7, Plum bark necrosis stem pitting-associated virus, Peach latent mosaic viroid, variants of Peach latent mosaic viroid responsible for calico of the peach, Plum pox virus, Citrus psorosis virus, Citrus variegation virus, Citrus tristeza virus, Olive latent virus-1, Olive latent virus-2, Olive latent ringspot virus, Olive latent*

*yellowing associated virus, Turnip mosaic virus, Alfalfa mosaic virus, Cucumber mosaic virus, Impatiens necrotic spot virus, Tomato spotted wilt virus, Pelargonium zonate spot virus, Lettuce mosaic virus, Cucumber green mottle mosaic virus, Bean yellow mosaic virus, Celery mosaic virus, Artichoke mottled crinkle virus, Artichoke latent virus* and *Artichoke Italian latent virus*, **characterised in that** it comprises PCR primers and/or cloned nucleotide sequences and/or nucleic probes.

**2.** Kit according to claim 1, **characterised in that** it comprises at least one pair of PCR primers chosen from Vdpr1/Vdpr2; HSP45A/HSP45B; RD1/RDAp; H28/BBoSe down; G23MET2U/G23MET2L; ASP1/ASP2; ASPn1/ASPn2: PPVHCPRO fwd/PPVHCPRO rev; FPLMVd-59/FPLMVd-60; FPLMVd-61/FPLMVd-58; cp1/cp2; Sn272/Asn502; CTV-CPfor/CTV-CPrev; AL1h/OL1c; OL2h/OL2c; OLRh/OLRc; OLYh/OLYc; TuMV fwd5'/TuMV rev 5'; CPAMV1/CPAMV2; INSV5'fwd/INSV5'rev; RNA2-5' fwd/RNA2-5' rev; BYMVCPses fwd/BYMVCP anti rev; TSWVNSM fwd/ TSWVNSM rev; PZSVcp fwd5'/PZSVcp rev5'; LeMV fwd5'/LeMV rev5'; CGMMV fwd5'/ CGMMV rev5'; AMCV fwd5'/AMCV rev5'; AILV fwd5'/AILV rev5' and ArLV fwd5'/ArLV rev5', having the sequences number (SEQ. ID NOS): 1-14, 16, 17, 19, 20, 22-25, 27, 28, 30, 31, 34, 35, 37, 38, 40-43, 45, 46, 48, 49, 51, 52, 54, 55, 57, 58, 60, 61, 63, 64, 66, 67, 69, 70, 72, 73, 75, 76, 78 and 79.

**3.** Kit according to claim 2, **characterised in that** said pairs of primers are used to synthesize fragments of bicatenary cDNA to be used for diagnostic purposes and/or for the preparation of recombining plasmids.

**4.** Kit according to claim 1, **characterised in that** it comprises at least one nucleotide sequence chosen from the following sequences (SEQ. ID NOS): 15, 18, 21, 26, 29, 32, 33, 36, 39, 44, 47, 50, 53, 56, 59, 62, 65, 68, 71, 74, 77, 80 and 81.

**5.** Kit according to claim 4, **characterised in that** said nucleotide sequences are contained in recombining plasmids.

**6.** Kit according to claim 5, **characterised in that** said recombining plasmids are chosen from: pASP3; pPPV-hc; pGPL1.1; pCPS600; pGEMCVVCP60x/3; pCTV.CP.2; pCTV.CP.0032; pSPTOLV1; pSPTOLV2; pGEMOLYV, pTuMV-cp-5; pAMV-Da; pINSV-NSP; pGEMCMV-2a; pGemTBYM.4; pGEMT-Dat2; pLeMV, pCGMMVcp,; pAM1; pGTAILV.cp.6 pAL37-2 and pCeMV8.

**7.** Kit according to any one of the claims from 1 to 6, **characterised in that** said primers and/or said nucleotide sequences and/or said recombining plasmids are used for the synthesis of DNA and/or RNA nucleic probes.

**8.** Kit according to any one of the claims from 1 to 7, **characterised in that** said primers and/or said nucleotide sequences and/or said recombining plasmids and/or said probes are used for the specific identification of the plant viruses and viroids listed in claim 1 among other plant viruses and viroids, in preparation of nucleic acid extracted from plant tissue or in purified preparations.

**9.** Diagnostic kit for ascertaining the state of plant health of nursery vegetable, flower and fruit productions **characterised in that** it comprises nucleic probes obtained from the pairs of primers listed in claim 2 and/or from the nucleotide sequences listed in claim 4.

**10.** Kit according to claim 9 **characterised in that** it comprises pre-soaked nylon membranes containing positive controls and negative controls.

**11.** Kit according to claim 10 **characterised in that** said positive controls comprise the plasmid DNA or DNA amplified from plasmid by PCR used for the transcription of the probe that is to be used for diagnosis and said negative controls comprise extract from a healthy plant.

**12.** Use of a kit according to any one of the claims from 1 to 11, for ascertaining the state of plant health of nursery vegetable, flower and fruit productions.

**13.** Use of a kit according to any one of the claims from 1 to 11, for ascertaining the state of plant health of nursery productions of vegetable plants.

**14.** Method for detecting and identifying plant viruses and/or viroids in nursery vegetable, flower and fruit plants, which contemplates the use of at least one diagnostic kit according to any one of the claims from 1 to 11.

**15.** Nucleotide sequence chosen from the sequences 21, 47, 53, 59, 68, 71, 81 listed in claim 4.

**16.** Pair of primers chosen from FPLMVd-61/FPLMVd-58; TuMV fwd5'/TuMV rev 5'; INSV5'fwd/INSV5' rev; BYMVCPses fwd/BYMVCP anti rev; LeMV fwd5'/LeMV rev5' and CGMMV fwd5'/CGMMV rev5'.

**17.** Nucleic probes obtained from the sequences listed in claim 15 and/or from the pair of primers listed in claim 16.

Fig. 1

SEQUENCE LISTING

<110> UNIVERSITA' DEGLI STUDI DI BARI

<120> DIAGNOSTIC KIT AND METHOD FOR DETECTING PHYTOVIRUS INFECTIONS IN PLANT PRODUCTION

<130> 05139D53

<150> IT/MI2004A/000258

<151> 2004-02-17

<160> 81

<170> PatentIn version 3.1

<210> 1

<211> 27

<212> DNA

<213> Artificial Sequence

<220>

<223> Primer

<400> 1
gcdaargcdg grcaaaccat mgcstgy 27

<210> 2

<211> 21

<212> DNA

<213> Artificial sequence

<220>

<223> Primer

<400> 2
raaytcsccs swraasckca t 21

<210> 3

<211> 21

<212> DNA

<213> Artificial Sequence

<220>

<223> Primer

<400> 3
gtatctyatg taccaacaga t 21

<210> 4

<211> 20

<212> DNA

<213> Artificial Sequence

<220>

<223> Primer

<400> 4
ggtatgaaca arttcaatgc 20

<210> 5

<211> 19

<212> DNA

<213> Artificial Sequence

<220>

<223> Primer

<400> 5
cycarcayaa rgtvaacga 19

<210> 6

<211> 21

<212> DNA

<213> Artificial Sequence

<220>

<223> Primer

<220>

<221> misc_feature

<222> (7)..(7)

<223> N=A+C+G+T

<400> 6
catgcangts agrggrccra a 21

<210> 7

<211> 22

<212> DNA

<213> Artificial Sequence

<220>

<223> Primer

<400> 7
gtgctaagaa cgtcttcaca gc 22

<210> 8

<211> 23

<212> DNA

<213> Artificial Sequence

<220>

<223> Primer

<400> 8
cgagtagccc ttcgtttagc cgc 23

<210> 9

<211> 23

<212> DNA

<213> Artificial Sequence

<220>

<223> Primer

<400> 9
aatgactgtg atgtcgcttt tac 23

<210> 10

<211> 24

<212> DNA

<213> Artificial Sequence

<220>

<223> Primer

<400> 10
taccactacc aggaggttta ttca 24

<210> 11

<211> 20

<212> DNA

<213> Artificial Sequence

<220>

<223> Primer

<400> 11
cggtagggct gtgactaccg 20

<210> 12

<211> 23

<212> DNA

<213> Artificial Sequence

<220>

<223> Primer

<400> 12
gtagtccgct ggtacgctac aag 23

<210> 13

<211> 20

<212> DNA

<213> Artificial Sequence

<220>

<223> Primer

<400> 13

acgaatccga gtttcgtcgc 20

<210> 14

<211> 20

<212> DNA

<213> Artificial Sequence

<220>

<223> Primer

<400> 14
aggcactact gacctgtagg 20

<210> 15

<211> 589

<212> DNA

<213> Gene Fragment

<400> 15
tcgaatgttc ctccaccgaa atcatacaca agagtgtaag aagtcgcacc accagtcttc 60

tccagtagag aacagaaacc tgcagcagtc ggttcattga tgactgcccg aacaccaatg 120

ccaagagcat tacaagcagt gtaaacaaag ctgcgcttaa atgaattgta gtccgctggt 180

acgctacaag aggcactact gacctgtagg tttgtttggg aaatagtgag ttgtaccaaa 240

cctttgatat acaagtatat caagtccaca acaggcatga cgcgagttaa atttccggac 300

acaggtccaa tcgagacatt atatttatcc acaatcacag tatattctgg tttgagtttg 360

cgacgaaact cggattcgtt tatgtgatta caaccaaccc aacgcttaat atctcgataa 420

acatccaaac cctcttcggt agtcacagcc ctaccgatgc aaaaagtccc atcttttcgc 480

aaccccagaa tagtcggtat gaatatagaa tccgactctt cagtgcaacg ttgctcttat 540

tagcagggga gaaacataca ctgctgaaag tagttccaaa atcaaaccc 589

<210> 16

<211> 23

<212> DNA

<213> Artificial Sequence

<220>

<223> Primer

<400> 16

gtgtgccaag cgataatccc ttg 23

<210> 17

<211> 23

<212> DNA

<213> Artificial Sequence

<220>

<223> Primer

<400> 17
tttgctatga acattgcacc tcc 23

<210> 18

<211> 893

<212> DNA

<213> Gene fragment

<400> 18
tttgctatga acattgcacc tccttttgct gtgggcaaat ccacatactt cgaatcacct 60

gagttaccaa caactaaatg attctttgtt ggacttatta tttcagactc tagtggtgta 120

ccgtcttcgt gtgtgacaca gcaacattgg tatacaaaat tttcccctcg cttgctagtg 180

cattcctttg agacctcaaa atgaatgcac tcttcaccca agagctgttt acgtagtgct 240

gccagattcg ttgacataac aaggttgcca atggccaatt tgcgaatacc tctaggattt 300

tcacgaacaa tatgacgtct gtagccctca ctaacatcga tcacatcgaa ataattccta 360

aagaagcgtt tggcatgata ctctctctgt ccccacacga aattgccatt agtatcaagt 420

tgattatcac acataagctg catattgacg tgtgcttttg atgagatttt gttcctgaaa 480

gcctttacag atccgctcct aatgttctct gtgcgattct tctgatatcg cgctagttcg 540

cgcagatgat ccgaggcttc tatgtagtct tgtgcactca tcataccacc cttaatgatt 600

aattcattga tcttgttgag atgggaaaaa ggtgcttctt tgcgctcacc tatcatgtgc 660

gtgatatcct tgaaagcttc ataattctga tttttcacgc gcatcaattc acgatacctc 720

tttagaaaag cgagaacatg tgaaaattgt ggatagttat ccatcactgt tttctcatgc 780

tgctcaatta ctgtcgaaaa tcgatcacgt atttcctgtt gtgacatgta agaatacttt 840

tgagcacatt gcagacatgt gatttttcca caagggatta tcgcttggca cac 893

<210> 19

<211> 26

<212> DNA

<213> Artificial Sequence

<220>

<223> Primer

<400> 19
cttacctcat tgcgaggtgc ttagcc 26

<210> 20

<211> 26

<212> DNA

<213> Artificial Sequence

<220>

<223> Primer

<400> 20
gtgggacttt tccttctgga accaag 26

<210> 21

<211> 337

<212> DNA

<213> Gene Fragment

<400> 21
cttacctcat tgcgaggtgc ttagcctttc tatcggaact gcagtgtccg aatagggcac 60

cccaaggtgg aggggctgag aggccacaac tctctcataa gtctgggctt agcccactga 120

tgagccgttg agatacggcg aaacttatgt gtgaaaagag tttcgtctca tttcagagac 180

tcatcagtgt gctaagcaca gactcttcat ccagaatcac ttctggaggg gaccgggttt 240

gaatcccggg tagacgtcgt aatccagttt ctacggcggt acctggatca caccccctc 300

ggaaccaacc gcttggttcc agaaggaaaa gtcccac 337

<210> 22

<211> 20

<212> DNA

<213> Artificial Sequence

<220>

<223> Primer

<400> 22
aaggtggagg ggctgagagg 20

<210> 23

<211> 22

<212> DNA

<213> Artificial Sequence

<220>

<223> Primer

<400> 23
atccaggtac cgccgtagaa ac 22

<210> 24

<211> 20

<212> DNA

<213> Artificial Sequence

<220>

<223> Primer

<400> 24
gcttcctgga aaagctgatg 20

<210> 25

<211> 22

<212> DNA

<213> Artificial Sequence

<220>

<223> Primer

<400> 25
tctgtttttg tcaacacact cc 22

<210> 26

<211> 592

<212> DNA

<213> Gene Fragment

<400> 26
tctggttttg tcaacacact ccatgtcgta gttgccattc accatcgcca aagaagtttg 60

tatcattctg aaacccctgg ttcttgtcgg ttattatcct gtcggccatg tcaattctgc 120

catgtggagt gaggctgtaa attatggcac atgttaactt cagagtgaag tttttattgt 180

tggtttttca tcagataaag gatgaagcct tttctgattc ttccattgtc cctccattgt 240

tttcagaaaa tctcttattg caattgattt ttcaatcttc tgattggttt ctagcaatgg 300

catcagggat tcaggagttg caccaacagc aggggataac ttttgcttgg tttgaaaact 360

gctcgcaaac ccagcatatc tcacagatcg attacctgca atattcagct tacacctaga 420

gcaaacagca cttggtaagt tgtctatatc aatcttgaga aacacccttg cagggaattt 480

ctttgttgga gccaattctt cactgatttg tgacaaactt acatcaccat catacaactt 540

cgatttgaca aattcaggta ctttcctttt gtcatcagct tttccaggaa gc 592

<210> 27

<211> 20

<212> DNA

<213> Artificial Sequence

<220>

<223> Primer

<400> 27
taccattgcc tacatgaccc 20

<210> 28

<211> 21

<212> DNA

<213> Artificial Sequence

<220>

<223> Primer

<400> 28
gccttcactt cggaaaccgt g 21

<210> 29

<211> 249

<212> DNA

<213> Gene Fragment

<400> 29
gccttcactt cggaaaccgt ggttccggta gggaaaacgt gctgtttagc acaatatttt 60
cctctcttca cacggatcct atcaggagcg acaggtccgg tggcattatt aacatcgaaa 120
ccatccataa acccggcatg tccatcggat ttgcatacga accctatcat caccgaatac 180
actttggtgg tatcatggag cgtttttatt ttacttagct ccgaacgcag ggtcatgtag 240
gcaatggta 249

<210> 30
<211> 19
<212> DNA
<213> Artificial Sequence

<220>
<223> Primer

<400> 30
atggacgacg aagacaaag 19

<210> 31
<211> 21
<212> DNA
<213> Artificial Sequence

<220>
<223> Primer

<400> 31
tcaacgtgtg ttagaatttc c 21

<210> 32
<211> 672
<212> DNA
<213> Gene Fragment

<400> 32
tcaacgtgtg ttaaatttcc caagctgcct gacattggta actacgactt catcagcccc 60
tcgcttcttc aacaattgtt ctttagcttg tatgtacaca gcacactcta aatcagtcaa 120
gccagctccg gtcaagaaat ctgcacacag gtaatgatac ccggccggaa tccctgcatc 180
tagcggacgt ccgccataac tcaaattgcg attctgtcta caaaaggcta aataaagggc 240

atcgttagtc ctaccccaga ctcgaagggc gttagcacgg ttaccgatac ccttagaatt 300
aaacacgacg tcagtccaaa gtttgtcaga caaatccact tcgacgccct cccgagtgta 360
cgttataccc gtagtgtcgt catcactttg caatgatgaa ctcttaaccg ctaaacgata 420
caacatcata gctatgtgaa agtccttatc tttatcagac aaattaggat acttcccttt 480
caaagtaaga aacaaatctc tgttcaaagc ggcattctgt tgggtaccca actgacgcac 540
atcgttcatc gttatcagag tcggatcgat gtgtaagttt acagaaccga aagaagactc 600
cgctgcaaca acattgtcgc cttctttcgt ttccttgttt ttgttcttca atttctttgt 660
ttcgtcgtcc at 672

<210> 33
<211> 672
<212> DNA
<213> Gene Fragment

<400> 33
tcaacgtgtg ttaaatttcc caagctgcct gacattagta actacaacct catcagcccc 60
tcgctttttc aacaattgtt ctttagcttg tatgtacaca gcacattcta aatcagtcaa 120
gccagctccg gtcaagaaat ctgcacacag ataatgatac ccagccggaa tccctgcatc 180
tagcggacgt ccgccataac tcaaattgcg gttctgtcta caaaaggcta agtaaagagc 240
atcgttagtt ctaccccaga ctcgaagggc gttagttcgg ttaccaatac ccttagaatt 300
atacacgacg ttggtccaaa gtttgtcaga caaatctact tcaacaccct cccgagtgta 360
cgttataccc gtggcgtcat catcactttg caatgatgaa ctcttaaccg ctaaacggta 420
taacatcata gctatgtgaa agtccttatc tttgtcaggc aagttaggat acttcccttt 480
cagagtaaga aataagtctc tgttcaaagc agtattctgt tgagtactca actgacgcac 540
atcgttcatc gttatcaaag tcggatcgat gtgtaagttt acggaactgt aagaagactc 600
agccgcaaca acatcgtcgc cttcatttgt ttcgttggtt ttgttcttca atttctttgt 660
ttcgtcgtcc at 672

<210> 34
<211> 19
<212> DNA
<213> Artificial Sequence

<220>
<223> Primer
<400> 34

tttcacccca ccaaatggc 19

<210> 35

<211> 19

<212> DNA

<213> Artificial Sequence

<220>

<223> Primer

<400> 35
ctcacccatc gttgtgtgg 19

<210> 36

<211> 1049

<212> DNA

<213> Gene Fragment

<400> 36
agcccctcaa tctggtgttg ggtccactac cccccgtccg agactttcgt cccgtcctcg 60

gcgtggatac ctgtgcagac cctcccatga gtcttgtaga ttgtatcagc aagtgatgcg 120

acggccgggg caccaccccc agcccgttaa acctgtctac caggatccga ttgcttaccg 180

ctctactaaa acttttaggg attagcacag ttacgtaact cacccatcgt tgtgtggtta 240

caaattgaca gttgggttca ctggttctat aaattcgatt tcatactggc agaacacctg 300

gccaaagttg gtggcagcgg ctggcccttg atcacttgca gtaaacaggg taactccgca 360

ggtatccgtt ctggatgttg gtgccatggc cagaaaggtg gccagggtaa cattagggta 420

ccacaacttg tccattcgag cgcagtccac gtccacgaca accattccag gaatgttgcc 480

gaatgacgct aaggccatag caccatcata ccccgcgtaa ggcggaaaag taatggcacg 540

gtaaccctgt tgcatttgag tgatgcttgt tggctgcgta tcgattctgt cgaatgatag 600

tgacatagaa acattgcctt gtgtgctggt tgggcaaacc ggtagataaa tgaaccggag 660

tttgcgccat cggtacttgc tgtacaaatc ggacaaacct gccaaccatg atagattgga 720

cgggattacc gtgacgtagt tgccgaaaat ggcacctaga gcggcagagt tgatggcact 780

tatgatttct gtgtttctca cgacagtctg ctcattagta gtagtaattc gaggaacgtg 840

cccttctgta gaaatcactc caccagcaac gggtgctata accggaagaa cttgtaggtt 900

gcccctattt gggggtgacc ttttagtctt gcgagcaggc agacttgcca tttggtgggg 960

tgaaatccgc tttatataag tgcggacacg ctgtggtcca ctttcgtaga accacctctg 1020

aattcgacta tcccagtaaa aattaacca 1049

<210> 37

<211> 22

<212> DNA

<213> Artificial Sequence

<220>

<223> Primer

<400> 37
acgtgttagt cgctgtggta cc 22

<210> 38

<211> 21

<212> DNA

<213> Artificial Sequence

<220>

<223> Primer

<400> 38
tatgtttgac gcaccggagc g 21

<210> 39

<211> 1279

<212> DNA

<213> Gene Fragment

<400> 39
gacggaggaa aatctccctc tgcacatcat gcaacagctt accttgagat ttatcaaatt 60

tactcagatc aatttctgtt aaacacttcc cctcaaagct aggccaaaca cgggagaagt 120

cagccatcgg accagccgga acaacaaccc aatcttgcag acgagagaaa aatcttgcca 180

tcacctgcag aaacatgggg gaaaaaacct cggtaaccca cttctcatga taagtaatgg 240

tttggggtac cgatcgaacc ggaaagttca gatctagaga gaatttccca gcggacttca 300

gcataactaa gtatgaggac aaggggtcat cgtaacaatt ctccaatgaa aagacctttc 360

gaccctttct atggaaaaaa tcatcccaat aatgaccatc cccctttatcc cagccgcctt 420

ctttcaagaa tgtccggaag aacctatcag ccaacctctt cacgtggatc tcctgaggta 480

cctctaccat ctgaggagct ccaacgtttc tcttcgagaa tgccattaac atttccttct 540

ttgtagcaat tcgaggcatc tcgagacctg tacgaatgga cgacacagcg aaatcgggtt 600

tccttctgaa ccaatcttcg tcccgttcgc gtaatcgcac ttgggtgggg aaatccacag 660
taaagtcatc agttccacct gccattgatc tgtggcaggt tcacgattaa aatgctcggg 720
tgccttgtct atgaaaaact gatcaattaa agcgtcatca cctcgcggga gacagaccct 780
agggactaag ggttccgatt gagctcgtgg caactcaacc tctttcagtt tctcaccgga 840
aaaatccgac cgagcgttca gaagaagggt gggattaaag tgttcaaaca attccgtacg 900
gactaatctc accatctcta catccattgg aggctcagga gccaaggctc tctcttgcgg 960
atgtacgaaa tgtacgtcag aaatcaatcc ttctggttcc caaaaatcca gagcagacac 1020
gggctcgacc tttctttcga tcttaatttc aatctcatga ggtattggca cgtatttcaa 1080
gaaacaacca gccaataatg ccaagcctag gtcttctgtt aaccatttca ctttcccgtc 1140
ttcgaactta aaggcactag aaatagtgtt cgtggcgtca aatgttagac cagttccacc 1200
tttggcacga tagatgaatt gcgtaccaca cgaacaacta caccttcctg ggtcacggc 1260
ataaaaagct tgcatgcag 1279

<210> 40
<211> 20
<212> DNA
<213> Artificial Sequence

<220>
<223> Primer

<400> 40
tgcaaaacta gtgccagagg 20

<210> 41
<211> 17
<212> DNA
<213> Artificial Sequence

<220>
<223> Primer

<400> 41
tgcataaggc tcaggag 17

<210> 42
<211> 19
<212> DNA
<213> Artificial Sequence

<220>

<223> Primer

<400> 42
tttcacccca ccaaatggc 19

<210> 43

<211> 19

<212> DNA

<213> Artificial Sequence

<220>

<223> Primer

<400> 43
ctcacccatc gttgtgtgg 19

<210> 44

<211> 381

<212> DNA

<213> Gene Fragment

<400> 44
cgaagagagc ggctgaaggc tcgttcatga tatgaaaaac cggtaatcca gtacgttcct 60

ccgtagtctt cataaagaat ctttgagtag aagtgtattg acttggtacg gaaataacta 120

aaccactaca gcgaaaattt cttaacttct caaaatccct cacaactaag gcaatgaaga 180

aagagacgat ttcgtggaga ttacgaagtc gaggctgccc aaaagtaggg tcaatcctca 240

gtgaaaagtg taccccagga caagtgacgt tgtacagagg tttaagtttg gcacgtctct 300

cagaaagcgt ttttgagttg actcctatcc aacgtttaag gtctctataa actgagaaac 360

cctgtcgacc gtaaccgtcc c 381

<210> 45

<211> 25

<212> DNA

<213> Artificial Sequence

<220>

<223> Primer

<400> 45
attcctgata cacgctccga gagca 25

<210> 46

<211> 16

<212> DNA

<213> Artificial Sequence

<220>

<223> Primer

<400> 46
gcgccacgca gtgctg 16

<210> 47

<211> 473

<212> DNA

<213> Gene Fragment

<400> 47
gcgccacgca gtgctgctgc tttcatctgg atgtgtgctt ctctcgcacg tattggagtt 60

ctagaagtca tttcatagaa atcaaacgcg tagcgagcta agctcatgtc ggttaaattg 120

cgctgaagac catatcgtgg catgtatggt cggtcttggt tacgctttac aatgtacgct 180

tcagctacgt cactgaaatg ggccattatc tgcctaaatg tgggtttggc gtggtcaatg 240

agcggtttga ttggaaattc cacctgatcg tcgccgtcca tcatcaccca cattccgttt 300

atatttgggg aagttccatt ctcaatgcac cagaccatta aaccattaag aatgatttgc 360

attttatcct ccgttagttc atagtcagcc atgacgccct caaaccatgt attgaactgc 420

ttctgtgttg agcgcgtgtt ggataagtct gtctgctctg gagtgtacag aat 473

<210> 48

<211> 19

<212> DNA

<213> Artificial Sequence

<220>

<223> Primer

<400> 48
tccatcatga gttcttcac 19

<210> 49
<211> 19
<212> DNA
<213> Artificial Sequence

<220>
<223> Primer

<400> 49
aggacttcat accttgacc 19

<210> 50
<211> 752
<212> DNA
<213> Gene Fragment

<400> 50
aggacttcat accttgacct taatccaccc agtggaggtc agcattaaat gactttagca 60
tcccaaatta atggggtacg tcaatgacga tcaagatcgt cagcttcgtc gagcagcccc 120
acagtaatca aactgcggag gggccctgct cgcgggaacg cctttctctc gacccaaact 180
tcgttgaatc ggtatgaggg atttttagat ccctcaggct gcgcgtcgaa gtccagacag 240
agggctacgg cataggaatg cttgaaattt tgattttgca ttcccgcatg ggtaggagct 300
gtgaagacca actcaaaccc gaacttctca ttagcgtgaa acacgccttg aggaaattcc 360
tgcatgggat taccatgcgc aacggcccta ccatcctcag tcgtcacgtc atcagtgaga 420
caaaaggtgc cggcatggga cggtgttatg gaaaacacca tcctgtaaat cagatcctct 480
tcgaggatcc gaggtcccgc gaaatccttc agaaaactgt agaggaatct cacgccgagc 540
ccattaaaag agctcagact cagaggggtc ccgaggcttt gccacacgca gcccgtctgt 600
ggcagtatag tattcgtcgg ttttacaacc gggactttca acgccggagg cttcggcagt 660
tgagctttgc gcaaggcagc atagttctga gaacgtttag taggtttacc agctttccca 720
ccagctttct tttgtgaaga actcatgatg ga 752

<210> 51
<211> 20
<212> DNA
<213> Artificial Sequence

<220>

<223> Primer

<400> 51
tggggaacaa catcttcggg 20

<210> 52

<211> 20

<212> DNA

<213> Artificial Sequence

<220>

<223> Primer

<400> 52
cagaagttgc agacattgcc 20

<210> 53

<211> 629

<212> DNA

<213> Gene fragment

<400> 53
cagaagttgc agacattgcc agtgatttta cagccaaagt gtttactgtc ctgttgttgg 60

tctgggattg gttaaaaaca ggcttaagtg tgtacagcca ttcatgaaca cttctggtag 120

gagaaagaac actgactttg cccatggatt ggttgtttga atacttgaca tcaaaaagga 180

cttctttgac acaagacaat gatcctttat ttgcagcttc aatgaaatta tttggaggga 240

agatgtcaga tttttcagac cgaatcttga agtaatttca ggcactgcta tttgctcttc 300

aagcattctg agcaactgag aggataacac tttgagatga ccttgatgtt taacaccgtt 360

tgtgtttgtt gtgttagatc tgaccagaat ttcgacaaca ttccctgaaa aggagaaatt 420

caatcaatgt catcaaaaat aggcacaaaa catctcacta taatttcttt ttcttctgtt 480

gggagaaagc caactttacc agtgtaacag aaactggtct tgctcctgga gtcagaatag 540

agttgagctt cgaccaactt ctttccggaa gattgatcat gaatccaata actatctact 600

actgctttac ccgaagatgt tgttcccca 629

<210> 54

<211> 22

<212> DNA

<213> Artificial Sequence

<220>

<223> Primer

<400> 54
gtttatttac aagagcgtac gg 22

<210> 55

<211> 23

<212> DNA

<213> Artificial sequence

<220>

<223> Primer

<400> 55
ggttcgaaag agatataacc ggg 23

<210> 56

<211> 637

<212> DNA

<213> Gene Fragment

<400> 56
ggttcgaaag aataaccggg tacatcgcga gacgagatat ctatctgagc gtcgtcggct 60

tcacactctt cactgtagta gaaatcagat ttagtgtaaa tagccgcgac caggtcttca 120

aaacacttca tggttcggtg ggatgttcta ggtgccaaca acaaagctct ggccatagca 180

atgctggaaa taaaagaaca tttattaaac gcagggcacc atagttgtcc gatacgcatg 240

ggtttgacca tcgtctcaaa agcgacaagg agctcatcat cagatttttc aaggtaatct 300

tcagcaccat aagttacaaa ctcatcagac accgcttcag cgggagctcc gtcaggagtt 360

cggagggaat gcgcgtcctc tgtgacactc tcgctgacat ccacagcggg taggggcctg 420

taattacgac aggccgcagc agcctcttcg cgttgctcag atcgcaaacg ttccacatcc 480

tcgggagtgt cgacaccgta actgccgttc aaaagattgg ctagtgagaa tgcgggggca 540

gggaaagcca tagaaaagat actagaaaga aaagatttaa gtctagggag ttttacaggg 600

gaggcagggg ttgaaccgta cgctcttgta aataaac 637

<210> 57

<211> 20

<212> DNA

<213> Artificial Sequence

<220>

<223> Primer

<400> 57
acacaagcac agtttgaagc 20

<210> 58

<211> 19

<212> DNA

<213> Artificial Sequence

<220>

<223> Primer

<400> 58
agatcaagct cacacgagg 19

<210> 59

<211> 700

<212> DNA

<213> Gene fragment

<400> 59
agatcaggct cacacgaggc aacctcacta actgcttacc ctgtcagagt agagagaatg 60

atacacatac tgaatttaaa atgctggagg tgaaacctca ctaatacata gtattaagta 120

atgtaacgcc aaattataga gaatttaaag acggatactc taaatacgaa caccaagcat 180

ggtgtgcata tcacgattga ctctcctgct gtgtgtctct ctgtgttctc ctcgtctgtt 240

ccaacattgc cataagtcca aataatctag ttgacttgcc cctaactgct gccgccttca 300

tttgcatgtg tgcttctcta gcacgcacag gagttcttga agtcagtttg tagaagtcaa 360

aagcatacta gccaaaccat aatcggtcaa gttcctttga agtccatacc gtggcatgta 420

ccttctgttg cattcctctt ttcaatgtag gcttctgcaa cttctgagaa atgcgacatt 480

atttggcgga atgttggctt tgcattgtcc aagatgggtt ttagagggta tgtcacctgt 540

tcctctccat ccatcattgt ccattcacct tgtaaatctc ctgatgtgca ttttctatgc 600

accacaccat aaggccattc aaaataattc ccatttgtga atcctcaacc tcatatgctt 660

gcttgacacc attgtaccac gcttcaaact gtgcttgtgt 700

<210> 60

<211> 21

<212> DNA

<213> Artificial Sequence

<220>

<223> Primer

<400> 60
gaaggaaaca tcttcctttg g 21

<210> 61

<211> 21

<212> DNA

<213> Artificial sequence

<220>

<223> Primer

<400> 61
cctcttcttc ttcaactgat c 21

<210> 62

<211> 679

<212> DNA

<213> Gene Fragment

<400> 62
```
cctcttcttc ttcaactgat ctctcaagat ttgagctcaa ttctttaagc tgttttttaa     60
tctgcttctc actgtttcct ttaggaatta tcagcttgca agcctcaatg aatgcttgag    120
atctagctct aatggctctg tttagaggta tgaccatgca acttttatct ttatcagctc    180
tgggtgaatc acaaaactct tttgtccaag aatacatgac actaccaaaa gaaacccccct   240
tcttgtattc ttggctgcac atcaaatgca gctgacagca gttttctgga gtgttattca    300
ttttcggaat agaccagttc agataaaaga caaaacatat aggatcagtt atagtcccct    360
gacccttcag aatgacttgc ttttcagatg gcatgttggg atcgaccaga gcaaccacaa    420
gttttcctgt agggtttggt atagtggggc agacccatat cacaatcctg gaaatcatca    480
tgtattgttt tctgctgtcc caagttggac agatcttgat gaccttattt gcgttttgct    540
tcccgttgcc aacaaagaga tcatttttcc agtttgagat atgatggttt gtatctacta    600
tcattcttgc agaaagatca taaccctctg attctgtgat agaatcagac tcataggttc    660
caaaggaaga tgttccttc                                                 679
```

<210> 63

<211> 18

<212> DNA

<213> Artificial Sequence

<220>

<223> Primer

<400> 63
atgcccccta agagacag 18

<210> 64

<211> 19

<212> DNA

<213> Artificial Sequence

<220>

<223> Primer

<400> 64
acagaggtat atactctgc 19

<210> 65

<211> 625

<212> DNA

<213> Gene Fragment

<400> 65
acagaggtat atactctgct tggccaatct tttcgtgctc tacgaaaatc ttgacagtag 60

cgaccggaga gttgttgccc actatagcat agtccgtagc aaacagatac accctcaatt 120

tagcaaggtg ttcaactgtc ttccccggat agtggtaaat aacatgccct ttaccttccc 180

tcccagtaat tctgactgca ttactaggaa aagaccccac agagggggcg acccatctat 240

cacccaaagc acaccaaaac ttgtgcgcct cagggacagt aggatcaagg agcacaacaa 300

cagcaactct atttaatctc aagttaggcg actcgagaag agcatccgga accgtaagac 360

tccgctctgc cgtagcgaac ccagtatgag tcccaaacgt tagagacgga tcaaacgtca 420

cctccgtggc ggtaaagcca gggatgagct tgagcctact ttcagattcc gtgatatcag 480

cccagctggt actgggagac ctttcaacag acatagagag cccggaaaat tcacgggcga 540

gcttagcgag tgcctgctgg cgactgcgcc tcgcgcggtt ttgtctagct ttgcgcgtct 600

cagtgttctg tctcttaggg ggcat 625

<210> 66

<211> 20

<212> DNA

<213> Artificial Sequence

<220>

<223> Primer

<400> 66
gtagacgcaa agcttgatgc 20

<210> 67

<211> 21

<212> DNA

<213> Artificial Sequence

<220>

<223> Primer

<400> 67
ttagtgcaac cctctcacgc c 21

<210> 68

<211> 836

<212> DNA

<213> Gene Fragment

<400> 68
ttagtgcaac cctctcacgc ctaagagagt atgcatattc tgatttacat ctgcggctgt 60

gtgcctctcc gtgttctctt cctgggttga accgcctcca tccattccaa acagtctgtt 120

ctgtgttccc actagagcag ctgccttcat ttgattgtgc gcctctcttg cccgatttgg 180

ggtcgctgat gttgtttcgt agaagtcgaa agcatagcga gctagcccca tatcgttcaa 240

gcctcgtagc cgtccgtatc gtggcatata cggtttcttc ttgtttctca tctcaatgta 300

cgcctcggct gcgtcactga agtgggccat tatctggcga aacgtgggtt tcgcgtgttc 360

gatgatgggt tttagagcat attctacttg ttcttcacca tccatcatca cccatgttcc 420

atttatttcg ggatgtcccc gttttctata caccaaacca tcaatccgtt cagaattaat 480

tgcatgccac tatcatccac atcgtagtca ttcttaaccc cgtcgtacca tgactcgtac 540

tgtttctggg ttgcacgtgt gttcgatatg tctctctggt ttggttcgta ctccagcaaa 600

tgatcaaggt ttaaagccac tttacctcgg atcattggta acttcatttt ctgtgtgatt 660

gcctttgtac gaggtatggt gtgtttacca tgtagccctg cgttgatgtc gtcatccttc 720

ttcatctgcc cagaaccact tcctttctct gtgatgatat tatcctttgg atccgctgag 780

ttcttttgct tatcatcagt tttactgcct tggcctgcat caagctttgc gtctac 836

<210> 69

<211> 18

<212> DNA

<213> Artificial Sequence

<220>

<223> Primer

<400> 69
cttacaatcc gatcacac 18

<210> 70

<211> 18

<212> DNA

<213> Artificial Sequence

<220>

<223> Primer

<400> 70
gctttcgagg tggtagcc 18

<210> 71

<211> 478

<212> DNA

<213> Gene Fragment

<400> 71
gctttcgagg tggtagcctc tgaccagact accgaaaacg cggcttcaaa tgaagcccta 60

tcgtaaacat caaaaccctt agaaatagac tctattaaat tatctatctc agccctagcg 120

gccgtagacg cgtcatcagt acgctttaca gcgttaagcg actcagcagt cgtaggattg 180

ctaggatcta caacctcaat gaccctatta cgcgtatccg tggagctgag aagcgaaacg 240

aagataggcc tcaacacagg accgttgagg aaagcgtaaa aacccgcatc tgggaatcta 300

gaattaatat ctacgacaga cgagggtaac gcagacaggg actcgcggaa agaatctctt 360

cccgcttgag tctggaaagc ggtaccttgt gaagcaacta gaaaattaag taaagtcctg 420

acgggaacat aagaagcact aaacgcaata agtttgctag gtgtgatcgg attgtaag 478

<210> 72

<211> 20

<212> DNA

<213> Artificial Sequence

<220>

<223> Primer

<400> 72
atggcaatgg taaagagaaa 20

<210> 73

<211> 26

<212> DNA

<213> Artificial Sequence

<220>

<223> Primer

<400> 73
ctcggacttt cgtcaggaag tttgaa 26

<210> 74

<211> 1349

<212> DNA

<213> Gene Fragment

<400> 74
ctcggacttt cgtcaggaag tttgaaccca gaagtggtac ctcctgatcc tccatcccaa 60

cgttcaccat tagcttgttc cctagcgtcg tttccttgta taactcgctc catggtttat 120

tgacttgttc gtattcagta tccatgagct ggtctagttc aagaggcccc tcacaggact 180

cactaaatta aagagagatc gttctgtcgc gtagcacgat tgcactgtat cgtggcagat 240

gttataccag tggtagtaaa cgtgataacg gacggtaggt ttgagacagt gcaatttatg 300

aaaaatgaac tgctcgttcc aacattgtcc acaactgtaa tgctgttcac gttgacccca 360

cctgtcaggc cgagcactgt tgaagttaga caacgatatg ctccagagat gacaaacgtg 420

ccagtggcac agatagtcat tgtaagtaca gtgggcgtcc tggttaacag gatgtacccg 480
ggtccggatg ctgtgaccgg tgtgccagca atgtcgagcc ttctagtgct aattagagta 540
ttcgtaggtt gagggaaata taacgtaaca ctgtaggaga tgaagatatc cccaacagca 600
ttagttccag cgccaccgta tgtagccacg cccaactgtc ctagatcgat aagtttgtga 660
tctaatgtgg aactgtcatc acaaaatctc ttaatcctat cggtaggaac ccagagcgca 720
cgttcagccc agggggctgt ctctgctagt acgctgtagt ttgccaactc aaccctatca 780
gcaggttcag gatcttccga atctttatca aagtacatag ccactcgccc cacctcagtg 840
gttgcgcaca gagggacata atgtagcaaa acgctgttga acgagtactg atcaaagttg 900
gagcgtatcg cgggcaacca tgagaacaaa gtaccattca acgggttgag ctgtaacaaa 960
ttgccaacaa ttcccccgtt tacctggaaa cctgtggaca tattcacttg tgataagtat 1020
tcacggtgcg taaccgtcac agacccagac gtcttaccgg taaacttagg cttactcccg 1080
gtaagttgtc tcgtaactgc aaccggggcc ataatggccc cacctgtacc acctacatgc 1140
ttaatctgct gctgtttctt acctcctcgc ttcttcactg ctttgtacac cttctgtcca 1200
acatcgacga cctttccgac aatagccgcc ccgttgttcc ttacaaatcc ctgcagggct 1260
gaagcccctg cggccgctcc taatgccatt aactgcttca tgctaactgg gataagccca 1320
gtgttattat ttctctttac cattgccat 1349

<210> 75

<211> 20

<212> DNA

<213> Artificial Sequence

<220>

<223> Primer

<400> 75
attcactagt ccctatttag 20

<210> 76

<211> 20

<212> DNA

<213> Artificial Sequence

<220>

<223> Primer

<400> 76
ggtctggggt gcccgtggcg 20

<210> 77

<211> 769

<212> DNA

<213> Gene Fragment

<400> 77
ggtctggggt gcccgtggcg cactcaatgg cataatccaa aagcgcgcac cactatcacc 60

aaaattggct gtggcacaac aggcagcttg gataacagct ctccagtcca gccccatcaa 120

tgtcaatgtg tggaatttgg gccgtctgca ctgcggttgg ctgtgttccc aaagaaaaac 180

acgaagctta cttgttaaca aacaactgga agtcgagtgt acacgaaacc tcagctttcc 240

cccaattccc aagaagtggg acaagagtcc tgcagagtag gaataggttt ccctctttgt 300

tgaagtaata gacttttcag caaaggaaag agggaaattg gtggtagcag ttgtaccaat 360

agcaagttgt tggggtgcca acagtagatc aagatccgcc aagtgcgtgg actcgacgga 420

tagggttaaa atctttctcc gtagcaagtc ctcggacgaa actttctcct gtggatatcc 480

ttcaacaaca tattgaacat ccgcggaaca cggcatttgg gccgaggagg cagcaatgat 540

ccaaaccttg ggcttcataa accctggtcc ggcaaagttc aaactctggc ccccctctct 600

gtggaaattg aggatccacg tgctggtagt attgtctttc aaaatgtgca cgtgtggact 660

caagtgacgg atgctttcca attccacagt tgtggtaacg tccgtgggaa tatggccata 720

agcatcaaaa acataccaga tggccattcc taaataggga ctagtgaat 769

<210> 78

<211> 20

<212> DNA

<213> Artificial Sequence

<220>

<223> Primer

<400> 78
ttgttcataa gggagcgcgt 20

<210> 79

<211> 20

<212> DNA

<213> Artificial sequence

<220>

<223> Primer

<400> 79
ctcaagctct cgaactaact 20

<210> 80

<211> 499

<212> DNA

<213> Gene Fragment

<400> 80
ctcaagctct cgaactaact gaactacaga tgactttagg gcaacgtcat ccactttgca 60

aaggtgttgg ggtctgttgt acttgagtaa atccttccaa tacgcttcac ggttcagtgc 120

gcttggcatg tagttgtcct catacttctc cacaacagga tcaacccatt cgttgcacaa 180

cttaaattgc ttccaatagg gattctctcc ttcaaagata tgcctcgtgt tcaacagccc 240

attggagtag gctaaggttg tgcatgtgta tcgctagatc agttggtggc aagttgcgtt 300

tttccaccac ctcgcgtggc ttaaacaagc caccccgtgc cgcacttctc attaagttct 360

caactgagta gacacttgtg tcacgtgtaa actgtctgaa ctcgattgtg tcaatctgca 420

agttcttaat gcgtgcagag ttgtgaccat gcatctcggg tacaaattcc caggccttaa 480

cgcgctccct tatgaacaa 499

<210> 81

<211> 400

<212> DNA

<213> Gene Fragment

<400> 81
atctcttgag ttaattgttg ataaccccaa gattccacca tcgctgcaca aatatcctcc 60

aatctatgag ctggttcact tgatcgatcc cattctagta ttgagacaat tctctcttcc 120

tcaagttttg gtatatatat ttctcccaca cgaactccct tatgtgacat gaaccataac 180

tcctccttgt tctcagttct ggtggtgaat gtgtatttga gtcccatctc tctgaaaatg 240

tcttctagct tgcttgcgat gtgttcatga tctggatgaa atgctacaag taagtcgtcc 300

ccatttatta ggaatctgca cacgtcgtcg tgtacatttt cctcatagcc aaggagcgtt 360

aacgagtacg tcattgccaa aaccaccatc aaagtattgt 400